(19) Europäisches Patentamt
European Patent Office
Office européen des brevets

(11) Veröffentlichungsnummer: **0 180 850**
**A2**

(12) # EUROPÄISCHE PATENTANMELDUNG

(21) Anmeldenummer: 85113443.7

(22) Anmeldetag: 23.10.85

(51) Int. Cl.[4]: C 07 D 249/08
C 07 D 233/60, A 61 K 31/41
A 61 K 31/415

(30) Priorität: 02.11.84 DE 3440115
13.07.85 DE 3525043
04.10.85 DE 3535456

(43) Veröffentlichungstag der Anmeldung:
14.05.86 Patentblatt 86/20

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Böckmann, Klaus, Dr.
Andreas-Gryphius-Strasse 7
D-5000 Köln 80(DE)

(72) Erfinder: Regel, Erik, Dipl.-Ing.
Untere Bergerheide 26
D-5600 Wuppertal(DE)

(72) Erfinder: Büchel, Karl Heinz, Prof. Dr.
Dabringhausener Strasse 42
D-5093 Burscheid(DE)

(72) Erfinder: Plempel, Manfred, Dr.
Zwengenberger Strasse 3c
D-5657 Haan(DE)

(54) **Antimykotische Azolylmethyl-cyclopropyl-carbinol Derivate.**

(57) Die neuen substituierten Azolylmethyl-cyclopropyl-carbinol-Derivate der Formel

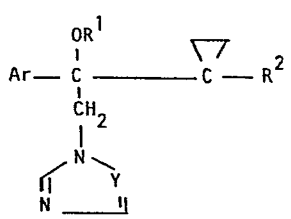

in welcher
Ar, $R^1$, $R^2$, $R^3$, X und Y die in der Beschreibung angegebene Bedeutung haben, können als Wirkstoffe antimykotischer Mittel verwendet werden.

EP 0 180 850 A2

Croydon Printing Company Ltd.

BAYER AKTIENGESELLSCHAFT 5090 Leverkusen, Bayerwerk
Konzernverwaltung RP Si/by-c
Patentabteilung

BEZEICHNUNG GEÄNDERT
siehe Titelseite

## Antimykotische Mittel

Die vorliegende Erfindung betrifft neue substituierte Azolylmethyl-cyclopropyl-carbinol-Derivate bei der Verwendung zur Behandlung von Krankheiten, insbesondere Mykosen.

Es ist bereits allgemein bekannt geworden, daß bestimmte Diazolyl-Derivate, wie beispielsweise 1,3-Di-(1,2,4-triazol-1-yl)-2-(3-chlorphenyl)- bzw. -(4-chlorphenyl)- bzw. -phenyl-2-propanol, antimykotische Eigenschaften aufweisen (vergleiche EP-OS 0 044 605). Die Wirkung dieser Stoffe ist jedoch nicht immer in allen Indikationsbereichen voll zufriedenstellend.

Es wurde gefunden, daß die neuen substituierten Azolylmethyl-cyclopropyl-carbinol-Derivate der Formel (I)

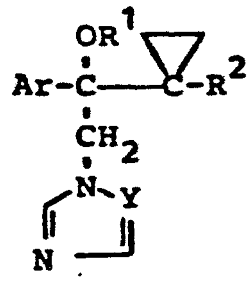

(I)

in welcher

Ar für gegebenenfalls substituiertes Aryl oder gegebenenfalls substituiertes Hetaryl steht,

$R^1$ für Wasserstoff, Alkyl, Alkenyl, Alkinyl, Trialkylsilyl, gegebenenfalls substituiertes Phenylalkyl oder den Acyl-Rest steht,

$R^2$ für Halogen, Cyano, Thiocyano, Alkylcarbonyloxy, Alkylcarbonylthio oder die Gruppierungen $-X-R^3$ und $-NR^4R^5$ steht, worin

$R^3$ für Alkyl, Cycloalkyl, Alkenyl, Alkinyl, Hydroxyalkyl, Alkylthioalkyl, Carboxyalkyl, Alkoxycarbonylalkyl, gegebenenfalls substituiertes Aryl, gegebenenfalls substituiertes Aralkyl oder den Rest der Formel

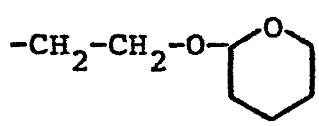

steht,

$R^4$ und $R^5$ unabhängig voneinander für Wasserstoff oder Alkyl stehen oder

$R^4$ und $R^5$ gemeinsam mit dem Stickstoffatom, an das sie gebunden sind, für einen gegebenenfalls substituierten cycloaliphatischen Ring stehen, der weitere Heteroatome enthalten kann,

X für Sauerstoff, Schwefel, eine SO- oder eine $SO_2$-Gruppe steht,

und

$R^2$ außerdem auch für Wasserstoff steht, wenn Ar für gegebenenfalls substituiertes Heteroaryl steht,

und

Y für Stickstoff oder eine CH-Gruppe steht,

sowie deren Säureadditions-Salze gute antimikrobielle, insbesondere antimykotische Eigenschaften, aufweisen.

Überraschenderweise zeigen die erfindungsgemäß zu verwendenden neuen substituierten Azolylmethyl-cyclopropyl-carbinol-Derivate der Formel (I) in bestimmten Indikationsbereichen ein besseres Wirkungsspektrum als die aus dem Stand der Technik bekannten Diazolyl-Derivate 1,3-Di-(1,2,4-triazol-1-yl)-2-(3-chlorphenyl)- bzw.

(4-chlorphenyl)- bzw. -phenyl-2-propanol, welches konstitutionell und wirkungsmäßig naheliegende Verbindungen sind.

Die erfindungsgemäßen substituierten Azolylmethyl-cyclopropyl-carbinol-Derivate sind durch die Formel (I) allgemein definiert. In dieser Formel stehen vorzugsweise

Ar für gegebenenfalls einfach oder mehrfach, gleich oder verschieden substituiertes Phenyl, wobei als Substituenten vorzugsweise genannt seien: Halogen, Alkyl, Alkoxy und Alkylthio mit jeweils 1 bis 4 Kohlenstoffatomen; Halogenalkyl, Halogenalkoxy und Halogenalkylthio mit jeweils 1 bis 2 Kohlenstoffatomen und 1 bis 5 gleichen oder verschiedenen Halogenatomen, wie Fluor- und Chloratomen; sowie jeweils gegebenenfalls durch Alkyl mit 1 oder 2 Kohlenstoffatomen und/oder Halogen substituiertes Phenyl oder Phenoxy; weiterhin für Naphthyl sowie für einen gegebenenfalls einfach oder mehrfach, gleich oder verschieden substituierten 5- bis 6-gliedrigen Heteroaromaten mit Stickstoff, Sauerstoff und/oder Schwefel als Heteroatome, wobei als Substituenten vorzugsweise die obengenannten Phenylsubstituenten in Frage kommen;

$R^1$ für Wasserstoff, geradkettiges oder verzweigtes Alkyl mit 1 bis 4 Kohlenstoffatomen, geradkettiges oder verzweigtes Alkenyl und Alkinyl mit jeweils 2 bis 4 Kohlenstoffatomen, Trialkylsilyl mit 1 bis 4

Kohlenstoffatomen in jedem Alkylteil, Alkylcarbonyl mit 1 bis 4 Kohlenstoffatomen im Alkylteil, sowie für gegebenenfalls einfach oder mehrfach, gleich oder verschieden substituiertes Phenylalkyl mit 1 bis 2 Kohlenstoffatomen im Alkylteil, wobei als Substituenten vorzugsweise die bei Ar bereits genannten Phenylsubstituenten in Frage kommen;

$R^2$ für Fluor, Chlor, Brom, Cyano, Thiocyano, Alkylcarbonyloxy mit 1 bis 4 Kohlenstoffatomen im Alkylteil, Alkylcarbonylthio mit 1 bis 4 Kohlenstoffatomen im Alkylteil, oder für die Gruppierungen $-X-R^3$ und $-R^4R^5$, worin

$R^3$ vorzugsweise für geradkettiges oder verzweigtes Alkyl mit 1 bis 18 Kohlenstoffatomen, Cycloalkyl mit 3 bis 8 Kohlenstoffatomen, geradkettiges oder verzweigtes Alkenyl mit 2 bis 18 Kohlenstoffatomen, geradkettiges oder verzweigtes Alkinyl mit 2 bis 18 Kohlenstoffatomen, Hydroxyalkyl mit 1 bis 18 Kohlenstoffatomen, Alkylthioalkyl mit 1 bis 6 Kohlenstoffatomen in der Alkylthiogruppe und 1 bis 6 Kohlenstoffatomen in der Alkylgruppe, Carboxyalkyl mit 1 bis 18 Kohlenstoffatomen im Alkylteil, Alkoxycarbonylalkyl mit 1 bis 6 Kohlenstoffatomen im Alkoxyteil und 1 bis6 Kohlenstoffatomen im Alkylteil, sowie für jeweils gegebenenfalls einfach oder mehrfach, gleichartig oder verschieden substituiertes Phenyl oder Phenylalkyl mit 1 oder 2 Kohlenstoffatomen im Alkylteil steht,

wobei als Substituenten jeweils vorzugsweise die bei Ar vorzugsweise genannten Phenylsubstituenten in Frage kommen, oder

$R^3$ für den Rest der Formel

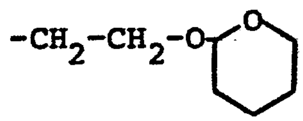

steht,

$R^4$ und $R^5$ unabhängig voneinander vorzugsweise für Wasserstoff oder geradkettiges oder verzweigtes Alkyl mit 1 bis 4 Kohlenstoffatomen stehen oder

$R^4$ und $R^5$ gemeinsam mit dem Stickstoffatom, an das sie gebunden sind, vorzugsweise für einen gegebenenfalls durch Alkyl mit 1 bis 4 Kohlenstoffatomen oder Alkylcarbonyl mit 1 bis 4 Kohlenstoffatomen im Alkylteil substituierten 5- oder 6-gliedrigen Ring stehen, der Sauerstoff, Schwefel und/oder Stickstoff als weitere Heteroatome enthalten kann, und

X vorzugsweise für Sauerstoff, Schwefel, eine SO- oder eine $SO_2$-Gruppe steht, und

$R^2$ außerdem auch für Wasserstoff steht, wenn Ar für einen gegebenenfalls substituierten 5- oder 6-gliedrigen Heteroaromaten steht, und

Besonders bevorzugt sind diejenigen Verbindungen der Formel (I), in denen

Ar für gegebenenfalls einfach bis dreifach, insbesondere einfach oder zweifach, gleich oder verschieden substituiertes Phenyl steht, wobei als Substituenten genannt seien: Fluor, Chlor, Methyl, Isopropyl, tert.-Butyl, Methoxy, Methylthio, Trifluormethyl, Trifluormethoxy, Trifluormethylthio, sowie jeweils gegebenenfalls durch Fluor, Chlor und/oder Methyl substituiertes Phenyl oder Phenoxy; weiterhin für Naphthyl sowie für jeweils gegebenenfalls einfach oder zweifach, gleich oder verschieden substituiertes Furyl, Thienyl, Pyridinyl oder Pyrimidinyl steht, wobei als Substituenten die oben genannten Phenylsubstituenten in Frage kommen;

$R^2$ für Wasserstoff, Methyl, Ethyl, n-Propyl, Isopropyl, n-Butyl, Isobutyl, Allyl, Propargyl, Trimethylsilyl, Methylcarbonyl, Ethylcarbonyl, n-Propyl-carbonyl, Isopropylcarbonyl, n-Butylcarbonyl, Isobutylcarbonyl, sowie für gegebenenfalls einfach bis dreifach, insbesondere einfach oder zweifach, gleichartig oer verschieden substituiertes Benzyl steht, wobei als Substituenten vorzugsweise die bei Ar bereits vorzugsweise genannten Phenylsubstituenten in Frage kommen,

$R^2$ für Fluor, Chlor, Brom, Cyano, Thiocyano, Methylcarbonyloxy, Ethylcarbonyloxy, n-Propylcarbonyloxy,

Isobutylcarbonyloxy, Methylcarbonylthio, Ethylcarbonylthio, n-Propylcarbonylthio, Isopropylcarbonylthio, n-Butylcarbonylthio oder für die Gruppierungen $-X-R^3$ oder $-NR^4R^5$ steht, worin

$R^3$ für geradkettiges oder verzweigtes Alkyl mit 1 bis 12 Kohlenstoffatomen, Cycloalkyl mit 5 bis 7 Kohlenstoffatomen, geradkettiges oder verzweigtes Alkenyl mit 2 bis 12 Kohlenstoffatomen, geradkettiges oder verzweigtes Alkinyl mit 2 bis 12 Kohlenstoffatomen, Hydroxyalkyl mit 1 bis 12 Kohlenstoffatomen, Alkylthioalkyl mit 1 bis 4 Kohlenstoffatomen in der Alkylthiogruppe und 1 bis 4 Kohlenstoffatomen in der Alkylgruppe, Carboxyalkyl mit 1 bis 12 Kohlenstoffatomen im Alkylteil, Alkoxycarbonylalkyl mit 1 bis 4 Kohlenstoffatomen im Alkoxyteil und 1 bis 4 Kohlenstoffatomen im Alkylteil, sowie für jeweils gegebenenfalls einfach bis dreifach, insbesondere einfach oder zweifach, gleichartig oder verschieden substituiertes Phenyl oder Benzyl steht, wobei als Substituenten die oben bei Ar bereits genannten besonder bevorzugten Phenylsubstituenten in Frage kommen, oder

$R^3$ für den Rest der Formel

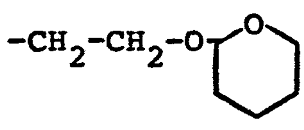

steht,

$R^4$ und $R^5$ unabhängig voneinander für Wasserstoff, Methyl, Ethyl, n-Propyl, Isopropyl, n-Butyl oder Isobutyl stehen oder

$R^4$ und $R^5$ gemeinsam mit dem Stickstoffatom, an das sie gebunden sind, für jeweils gegebenenfalls durch Methyl, Ethyl, Methylcarbonyl oder Ethylcarbonyl substituiertes Piperidinyl, Piperazinyl oder Morpholinyl stehen, und

X für Sauerstoff, Schwefel, eine SO- oder eine $SO_2$-Gruppe steht, und außerdem

$R^2$ auch für Wasserstoff steht, wenn Ar für einen der oben genannten gegebenenfalls substituierten Heteroaromaten steht, und

Y für Stickstoff oder ein CH-Gruppe steht.

Bevorzugte erfindungsgemäße Verbindungen sind auch Additionsprodukte aus Säuren und denjenigen substituierten Azolylmethyl-cyclopropyl-carbinol-Derivaten der Formel (I), in denen Ar, $R^1$, $R^2$ und Y die Bedeutungen haben, die bereits vorzugsweise für diese Reste genannt wurden.

Zu den Säuren, die addiert werden können, gehören vorzugsweise Halogenwasserstoffsäuren, wie z.B. die Chlorwasserstoffsäure und die Bromwasserstoffsäure, insbe-

sondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren wie z.B. p-Toluolsulfonsäure, 1,5-Naphthalindisulfonsäure oder Camphersulfonsäure.

Die erfindungsgemäß zu verwendenden substituierten Azolylmethyl-cyclopropyl-carbinol-Derivate sowie ihre Säureadditionssalze sind noch nicht beschrieben. Sie können in allgemein bekannter Art und Weise erhalten werden, indem man

a) in einer ersten Stufe Aryl-Cyclopropyl-ketone der Formel (II)

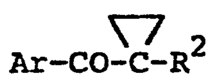

(II)

in welcher

Ar und $R^2$ die oben angegebene Bedeutung haben,

mit Dimethyloxosulfonium-methylid der Formel (III)

$$(CH_3)_2 \overset{\delta+}{SO} \overset{\delta-}{CH_2} \qquad (III)$$

in Gegenwart eines Verdünnungsmittels, wie beispielsweise Dimethylsulfoxid, bei Temperaturen zwischen 20°C und 80°C umsetzt (vgl. hierzu J. Am. Chem. Soc. 87, 1363-1364 (1965)); und die dabei entstehenden Aryl-cyclopropyl-oxirane der Formel (IV)

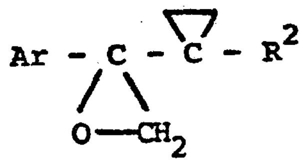

(IV)

in welcher

Ar und $R^2$ die oben angegebene Bedeutung haben,

in einer zweiten Stufe mit Azolen der Formel (V)

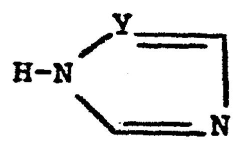

(V)

in welcher

Y die oben angegebene Bedeutung hat,

in Gegenwart eines inerten organischen Lösungsmittels, wie beispielsweise Acetonitril oder Dimethylformamid, und in Gegenwart einer Base, wie beispielsweise Kaliumcarbonat oder Kaliumhydroxid, bei Temperaturen zwischen 60 und 150°C umsetzt, oder

b) Azolyl-keto-Verbindungen der Formel (VI)

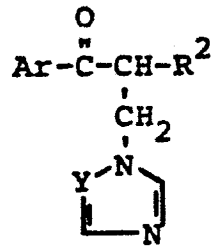

(VI)

in welcher

$R^2$, Ar und Y die oben angegebene Bedeutung haben,

mit Dimethyloxosulfoniummethylid der Formel (III)

$$(CH_3)_2 {}^{\delta+}SO^{\delta-}CH_2 \qquad (III)$$

in Gegenwart eines Verdünnungsmittels wie beispielsweise Dimethylsulfoxid, bei Temperaturen zwischen 20°C und 80°C umsetzt (vgl. hierzu J. Amer. Chem. Soc. 87, 1363-1365 (1965)); und gegebenenfalls

c) die nach dem Verfahren (a) oder (b) erhaltenen Azolylmethylthiocyclopropyl-carbinol-Derivate der Formel (Ia)

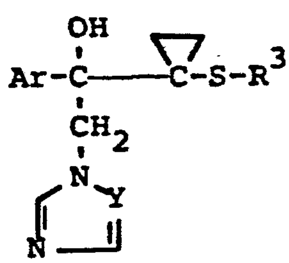

(Ia)

in welcher

Ar, $R^3$ und Y die oben angegebene Bedeutung haben,

nach bekannten Methoden in üblicher Weise oxidiert, wobei bei der Anwendung von 1 Mol Oxidationsmittel, wie m-Chlorperbenzoesäure in Methylenchlorid oder Wasserstoffperoxid in Essigsäure oder Acetanhydrid bei Temperaturen zwischen -30°C bis +30°C, vorzugsweise die erfindungsgemäßen Verbindungen der Formel (I) mit der -SO-Gruppierung entstehen; und bei Überschuß an Oxidationsmittel und höheren Temperaturen (10 bis 80°C) vorzugsweise die erfindungsgemäßen Verbindungen der Formel (I) mit der $-SO_2$-Gruppierung entstehen; und gegebenenfalls

d) die nach den Verfahren (a), (b) oder (c) erhaltenen Hydroxyverbindungen der Formel (Ib)

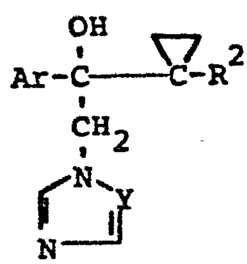

(Ib)

in welcher

Ar, $R^2$ und Y die oben angegebene Bedeutung haben,

in Gegenwart eines Verdünnungsmittels in das

Alkoholat überführt und dieses mit einem Halogenid der Formel (VII)

R-Hal (VII)

in welcher

R für Alkyl, Alkenyl, Alkinyl, Trialkylsilyl, gegebenenfalls substituiertes Phenylalkyl oder den Acyl-Rest steht und

Hal für Halogen steht,

in Gegenwart eines inerten organischen Lösungsmittels, wie beispielsweise Ether oder chlorierte Kohlenwasserstoffe, bei Temperaturen zwischen 20 und 100°C umsetzt.

In einer bevorzugten Ausführungsform des Verfahrens (d) wird zweckmäßigerweise so verfahren, daß man von einer Hydroxy-Verbindung der Formel (Ib) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einem Halogenid der Formel (VI) umsetzt, wobei unter Austritt von Alkalihalogenid die erfindungsgemäßen Verbindungen der Formel (I) in einem Arbeitsgang erhalten werden.

Nach einer weiteren bevorzugten Ausführungsform des Verfahrens (d) werden zweckmäßigerweise die Herstellung der

Alkoholate sowie die Alkylierung in einem Zweiphasensystem, wie beispielsweise wäßrige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt, wobei in der organischen Phase oder an der Grenzfläche die Alkoholate und mit den in der organischen Phase befindlichen Halogeniden umgesetzt werden.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Aryl-cyclopropyl-ketone der Formel (II) sind noch nicht bekannt. Sie werden erhalten, indem man Arylhalogenpropyl-ketone der Formel (VIII)

$$Ar-CO-\underset{\displaystyle Hal'}{CH}-CH_2CH_2-Hal'' \qquad (VIII)$$

in welcher

Ar die oben angegebene Bedeutung hat,

Hal' für Fluor, Chlor oder Brom steht und

Hal" für Brom oder Chlor steht,

δ) mit Verbindungen der Formel

$$HR^6 \quad (IX)$$

in welcher

$R^6$ für Cyano, Thiocyano, Alkylcarbonyloxy, Alkylcarbonylthio oder die Gruppierungen $-XR^3$ und $-NR^4R^5$ steht, worin

$R^3$ für Alkyl, Cycloalkyl, Alkenyl, Alkinyl, Hydroxyalkyl, Alkylthiaalkyl, Carboxyalkyl, Alkoxycarbonylalkyl, gegebenenfalls substituiertes Aryl, gegebenenfalls substituiertes Aralkyl oder den Rest der Formel

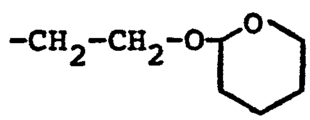

steht,

$R^4$ und $R^5$ unabhängig voneinander für Wasserstoff oder Alkyl steht oder

$R^4$ und $R^5$ gemeinsam mit dem Stickstoffatom, an das sie gebunden sind, für einen gegebenenfalls substituierten cycloaliphatischen Ring stehen, der weitere Heteroatome enthalten kann, und

X für Sauerstoff, Schwefel, eine SO- oder eine $SO_2$-Gruppe steht,

in Gegenwart eines Verdünnungsmittels und in Gegenwart einer Base umsetzt, oder

ß) direkt in Gegenwart eines Verdünnungsmittels und in Gegenwart einer Base erhitzt (wobei Keton der Formel (II) mit $R^6$ = Halogen erhalten werden.

Als Verdünnungsmittel kommen für die Herstellung der Ketone der Formel (II) gemäß Varianten (α) und (ß) unter den Reaktionsbedingungen inerte organische Lösungsmittel in Frage. Hierzu gehören vorzugsweise Alkohole, wie Methanol, Ethanol, Methoxyethanol, Propanol oder t-Butanol; Ketone, wie Aceton und 2-Butanon; Nitrile, wie Acetonitril; Ester, wie Essigester; Ether, wie Dioxan; aromatische Kohlenwasserstoffe, wie Benzol und Toluol; oder Amide, wie Dimethylformamid.

Als Basen kommen für die Umsetzung gemäß Varianten (α) und (ß) alle üblicherweise verwendbaren anorganischen und organischen Basen in Betracht. Hierzu gehören vorzugsweise Alkalicarbonate, wie Natrium- und Kaliumcarbonat; Alkalihydroxide, wie Natrium- und Kaliumhydroxid; Alkalialkoholate, wie Natrium- und Kaliummethylat, -ethylat und -tert.-butylat; Alkalihydride, wie Natriumhydrid; sowie niedere tertiäre Alkylamine,

Cycloalkylamine und Aralkylamine, wie insbesondere Triethylamin.

Die Reaktionstemperaturen können bei der Durchführung der Varianten (α) und (ß) in einem größeren Bereich variiert werden. Im allgemeinen arbeitet man bei Temperaturen zwischen 0 und 200°C, vorzugsweise zwischen 20 und 150°C.

Bei der Durchführung der Umsetzung gemäß Variante (α) setzt man vorzugsweise auf 1 Mol Keton der Formel (VIII) 1 bis 2 Mol an Verbindung der Formel (IX) und 1 bis 2 Mol Base ein. Die Isolierung dieser Zwischenprodukte erfolgt in allgemein üblicher Weise.

Die Aryl-halogenpropyl-ketone der Formel (VIII) sind bekannt (vgl. z.B. DE-OS 25 21 104, DE-OS 23 20 355 und DE-OS 23 51 948); bzw. können sie in allgemein üblicher Weise erhalten werden.

Die Aryl-halogenpropyl-ketone der Formel (VIII) mit Hal' = Fluor können z.B. durch Umsetzung der entsprechenden Ketone mit Hal' = Brom mit Alkalifluoriden in Gegenwart eines inerten organischen Lösungsmittels, wie z.B. Benzol, und in Gegenwart eines makrocyclischen Ethers, wie z.B. 18-Krone-6 (1,4,7,10,13,16-Hexaoxacyclooctadekan der Formel

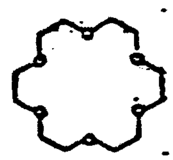 )

bei der Siedetemperatur des jeweils verwendeten Lösungsmittels erhalten werden (vgl. auch die Herstellungsbeispiele).

Die Aryl-cyclopropyl-ketone der Formel (II) stellen allgemein interessante Zwischenprodukte dar.

Die Verbindungen der Formel (IX) sind allgemein bekannte Verbindungen der organischen Chemie.

Das außerdem für die erste Stufe des erfindungsgemäßen Verfahrens (a) sowie für das erfindungsgemäße Verfahren (b) als Ausgangsstoff benötigte Dimethyloxosulfonium-methylid der Formel (III) ist bekannt (vgl. J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Die außerdem für die zweite Stufe des erfindungsgemäßen Verfahrens (a) als Ausgangsstoffe benötigten Azole der Formel (V) sind allgemein bekannte Verbindungen der organischen Chemie.

Die bei dem erfindungsgemäßen Verfahren (a) als Zwischenprodukte auftretenden Aryl-cyclopropyl-oxirane der Formel (IV) sind noch nicht bekannt. Sie stellen allgemein interessante Zwischenprodukte dar.

Die für das erfindungsgemäße Verfahren (b) als Ausgangsstoffe zu verwendenden Azolyl-keto-Verbindungen sind durch die Formel (VI) allgemein definiert. In dieser Formel (VI) stehen $R^2$, Ar und X vorzugsweise für diejenigen Reste die bereits im Zusammenhang mit der Beschreibung der erfindungsgemäßen Stoffe der Formel (I) als bevorzugt für diese Substituenten genannt wurden.

Die Azolyl-keto-Verbindungen der Formel (VI) sind noch nich bekannt. Man erhält sie, wenn man Ketone der Formel (X)

$$Ar-\overset{O}{\overset{\|}{C}}-CH_2-R^2 \qquad (C)$$

in welcher

Ar und R  die oben angegebene Bedeutung haben,

mit Hydroxymethylazolen der Formel (XI)

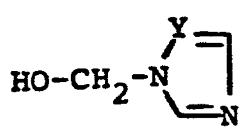

(XI)

in welcher

Y die oben angegebene Bedeutung hat,

in Gegenwart eines Verdünnungsmittels und in Gegenwart eines Katalysators umsetzt.

Als Verdünnungsmittel kommen für dieses Verfahren zur Herstellung der Azolyl-keto-Derivate der Formel (VI) vorzugsweise inerte organische Lösungsmittel in Frage. Hierzu gehören vorzugsweise Alkohole, wie Methanol und Ethanol; Ether, wie Tetrahydrofuran und Dioxan; aromatische Kohlenwasserstoffe, wie Benzol und Toluol; sowie halogenierte aliphatische und aromatische Kohlenwasserstoffe, wie Methylenchlorid, Chloroform, Chlorbenzol und Dichlorbenzol.

Dieses Verfahren wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen wie Pyridin und Piperidin; sowie insbesondere Piperidinacetat.

Die Reaktionstemperaturen können bei der Durchführung dieses Verfahrens in einem größeren Bereich variiert werden. Im allgemeinen arbeitet man zwischen 20 und 160°C, vorzugsweise bei der Siedetemperatur des jeweiligen Lösungsmittels.

Bei der Durchführung dieses Verfahrens setzt man auf 1 Mol Keton der Formel (X) 1 bis 1,5 Mol Hydroxymethylazol der Formel (XI) und katalytische bis 0,2-molare Mengen an Katalysator ein.

Die Ketone der Formel (X) sind allgemein bekannte Verbindungen der organischen Chemie oder lassen sich nach prinzipiell bekannten Verfahren in analoger Weise herstellen.

Die Hydroxymethylazole der Formel (XI) sind ebenfalls bekannt (EP 000 6102 sowie Chem. Heterocycl. Comp. 1980, 189).

Die Azolyl-keto-Derivate der Formel (VI) stellen interessante Zwischenprodukte dar und zeigen in entsprechenden Aufwandmengen bzw. -konzentrationen auch antimykotische, fungizide und pflanzenwachstumsregulierende Eigenschaften.

Die für das erfindungsgemäße Verfahren (b) als Ausgangsstoffe zu verwendenden Azolylmethyl-thiocyclopropyl-carbinol-Derivate der Formel (Ia) sind erfindungsgemäße Verbindungen.

Die für das erfindungsgemäße Verfahren (c) als Ausgangsstoffe zu verwendenden Hydroxyverbindungen der Formel (Ib) sind ebenfalls erfindungsgemäße Verbindungen. Ihre Überführung in die entsprechenden Alkoholate erfolgt in allgemein bekannter Art und Weise, indem man mit geeigneten starken Basen, wie Alkalimetall-amiden oder -hydriden, quarternären Ammoniumhydroxiden oder Phosphoniumhydroxiden in einem indifferenten Lösungsmittel, wie beispielsweise Dioxan, bei Raumtemperatur umsetzt.

Die außerdem für das erfindungsgemäße Verfahren (d) als Ausgangsstoffe zu verwendenden Halogenide sind durch die Formel (VII) allgemein definiert. In dieser Formel steht R vorzugsweise für die Bedeutungen, die bereits im Zusammenhang mit der Beschreibung der erfindungsgemäßen Stoffe der Formel (I) für den Substituenten $R^1$ genannt wurden, mit Ausnahme der Bedeutung von Wasserstoff.

Die Halogenide der Formel (VII) sind allgemein bekannte Verbindungen der organischen Chemie.

Die erfindungsgemäß verwendbaren Verbindungen der Formel (I) und ihre Säureadditions-Salze weisen antimikrobielle insbesondere starke antimykotische Wirkungen auf. Sie besitzen ein sehr breites antimykotisches Wirkungsspektrum, insbesondere gegen Dermatophyten und Sproßpilze sowie bi-phasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichphyton-Arten, wie Trichophyton mentagrophytes, Microsporon-Arten, wie Microsporon felineum sowie Torulospsis-Arten, wie Torulospsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

0180850

Dermatomykosen und Systemmykosen durch Trichophyton mentagrophytes und andere Trichophytonarten, Microsporonarten, Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tablette, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Mengen Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfest oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenen Überzügen und Hüllen versehen sein und so zusammengesetzt sein, daß sie den oder die Wirkstoffe

nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentione, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Oele, insbesondere Baumwollsaatoel, Erdnußöl, Olivenöl, Ricinusöl und Sesamöl, Glycerin, Glycerinformal, Tetrahydrofurfurylalkohol, Polyethylenglykole und Fettsäureester des Sorbitan oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oraler Applikation werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den ganannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben aufgeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

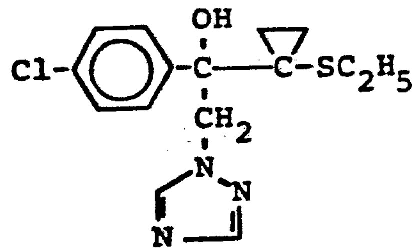

(I-1)

(Verfahren a)

1. Stufe

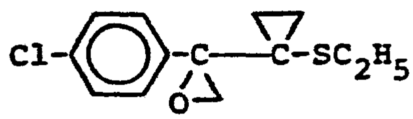

(IV-1)

Zu einer Mischung aus 6,4 g Natriumhydrid (80 %ig) und 44,2 g Trimethylsulfoxoniumiodid tropft man bei 10°C 170 ml trockenes Dimethylsulfoxid zu und läßt eine Stunde bei Raumtemperatur nachrühren. Anschließend versetzt man tropfenweise mit 40 g (0,167 Mol) 1-(4-Chlorbenzoyl)-1-ethylthio-cyclopropan in 50 ml Dimethylsulfoxid. Das Reaktionsgemisch wird zwei Tage bei Raumtemperatur gerührt. Danach gießt man auf 500 g Eis, extrahiert mehrmals mit Essigester, wäscht die

vereinigten organischen Phasen mit Wasser, trocknet über Natriumsulfat und engt ein. Man erhält 40,5 g (95,5 % der Theorie) rohes 1-/1-(4-Chlorphenyl)-oxiranyl/-1-ethylthio-cyclopropan als Öl, das direkt weiter umgesetzt wird.

2. Stufe

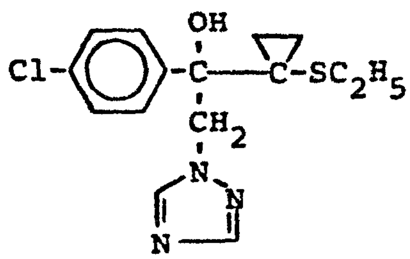

(I-1)

Zu einer siedenden Mischung aus 33,5 g 1,2,4-Triazol und 22 g Kaliumcarbonat in 150 ml Acetonitril tropft man eine Lösung von 40,5 g (0,16 Mol) rohes 1-/1-(4-Chlorphenyl)-oxiranyl/-1-ethylthio-cyclopropan (Beispiel IV-1) in 50 ml Acetonitril und erhitzt das Reaktionsgemisch 8 Stunden unter Rückfluß. Man läßt abkühlen, versetzt mit 400 ml Wasser und saugt das kristalline Produkt ab. Nach dem Umkristallisieren aus Ethanol/Wasser erhält man 26,5 g (51 % der Theorie) 1-(4-Chlorphenyl)-1-/1-(ethylthio)-1-cyclopropyl/-2-(1,2,4-triazol-1-yl)-1-ethanol vom Schmelzpunkt 165°C.

Herstellung des Ausgangsproduktes

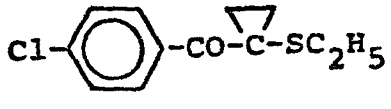

(II-1)

Zu einer Lösung von 100 g (0,338 Mol) 4-Chlorphenyl-(1-brom-3-chlorpropyl)-keton und 21 g Ethylthiol in 200 ml Dimethylformamid tropft man bei 0°C bis 10°C eine Lösung von 38 g Kaliumhydroxid in 250 ml Methanol. Man läßt eine Stunde bei Raumtemperatur und eine Stunde unter Rückfluß nachrühren. Anschließend wird im Vakuum eingeengt und der Rückstand in einem Gemisch von Wasser und Methylenchlorid aufgenommen. Die organische Phase wird abgetrennt, mit Wasser gewaschen, über Natriumsulfat getrocknet und im Vakuum eingeengt. Der Rückstand wird im Hochvakuum destilliert. Man erhält 70,1 g (86 % der Theorie) 1-(4-Chlorbenzoyl)-1-ethylthio-cyclopropan vom Schmelzpunkt $K_{p\ 0,15}$ = 127°C.

Beispiel 2

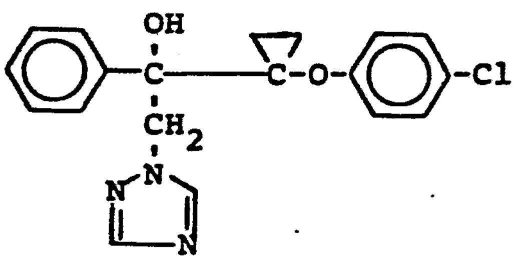

(I-2)

(Verfahren b)

19,7 g (0,06 Mol) 1-(4-Chlorphenoxy)-2-(1,2,4-triazol-1-yl)-propiophenon werden portionsweise eingetragen in eine Lösung von Dimethylsulfoxoniummethylid in 70 ml Dimethylsulfoxid (hergestellt aus 27,6 g (0,125 Mol) Trimethylsulfoxoniumiodid und 14,03 g (0,125 Mol) Kalium-tert.-butylat). Das Gemisch wird 6 Stunden bei

60°C gerührt, danach mit 1000 ml Wasser verdünnt, das abgeschiedene Öl in Chloroform aufgenommen, über Natriumsulfat getrocknet, eingeengt und chromatographisch (Kieselgel F60 Merck/Laufmittel : Chloroform) gereinigt. Das verbleibende Öl kristallisiert beim Verrühren mit Acetonitril. Man erhält 1,7 g (8 % der Theorie) an 1-(4-Chlorphenoxy)-1-/1-hydroxy-1-phenyl-2-(1,2,4-triazol-1-yl)7-cyclopropan vom Schmelzpunkt 170°C.

Herstellung des Vorproduktes

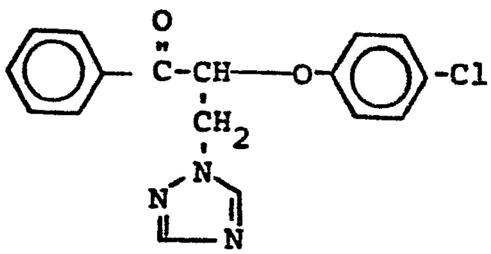

(IV-1)

29,4 g (0,12 Mol) -(4-Chlorphenoxy)-acetophenon und 11,9 g (0,12 Mol) 1,2,4-Triazol-1-yl-methylalkohol in 200 ml Toluol werden nacheinander mit 10,8 g (0,18 Mol) Essigsäure und 1,8 ml (0,018 Mol) Piperidin versetzt und am Wasserabscheider gekocht, bis die abgeschiedene Wassermenge konstant bleibt. Die Toluollösung wird anschließend mit Wasser gewaschen, über Natriumsulfat getrocknet, eingeengt und der ölige Rückstand chromatographisch gereinigt (Kieselgel F60 Merck/Laufmittel Chloroform). Man erhält 20,8 g (53 % der Theorie) an 1-(4-Chlorphenoxy)-2-(1,2,4-triazol-1-yl)-propiophenon vom Schmelzpunkt 126°C.

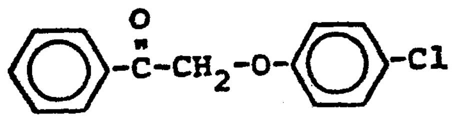

Eine Lösung von 154,6 g (1 Mol) ω-Chloracetophenon in 250 ml Butanon wird in eine siedende Lösung von 129 g (1 Mol) 4-Chlorphenol, 140 g (1 Mol) Kaliumcarbonat und 2 g Kaliumiodid in 800 ml Butanon getropft und das Gemisch wird nach beendeter Zugabe weitere 12 Stunden am Sieden gehalten. Die erhaltene Reaktionsmischung wird filtriert und eingedampft. Man erhält 179 g (73 % der Theorie) an -(4-Chlorphenoxy)-acetophenon vom Schmelzpunkt 97°C.

Beispiel 3

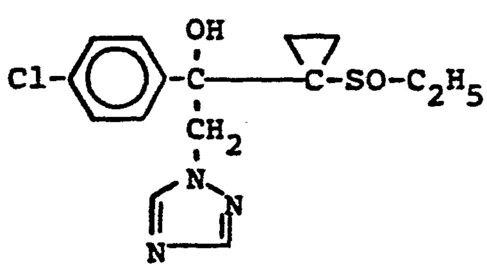

(I-3)

(Verfahren c)

5 g (0,0155 Mol) 1-(4-Chlorphenyl)-1-[1-(ethylthio)-1-cyclopropyl]-2-(1,2,4-triazol-1-yl)-1-ethanol (Beispiel 1)

werden mit 3,3 g m-Chlorbenzoesäure (80 bis 90 %ig) in 40 ml Methylenchlorid bei Raumtemperatur über Nacht und anschließend eine Stunde unter Rückfluß gerührt. Anschließend wird zweimal mit je 20 ml 5 %iger Natronlauge und zweimal mit Wasser gewaschen. Die organische Phase wird über Natriumsulfat getrocknet und im Vakuum eingeengt. Der Rückstand wird aus Glykolmonomethylether-acetat umkristallisiert. Man erhält 3 g (57 % der Theorie) 1-(4-Chlorphenyl)-1-/1-(ethylsulfinyl)-1-cyclopropyl7-2-(1,2,4-triazol-1-yl)-1-ethanol vom Schmelzpunkt 185°C.

Beispiel 4

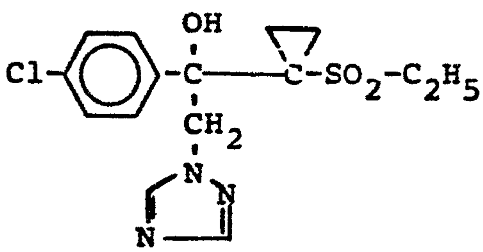

(I-4)

(Verfahren c)

3,2 g (0,01 Mol) 1-(4-Chlorphenyl)-1-/1-(ethylthio)-1-cyclopropyl7-2-(1,2,4-triazol-1-yl)-1-ethanol (Beispiel 1) werden in 20 ml Eisessig gelöst und mit 4 ml 30 %igem Wasserstoffperoxid sechs Stunden bei 70°C gerührt. Anschließend versetzt man mit 100 ml Eiswasser, neutralisiert mit konzentrierter Natronlauge und saugt den kristallinen Feststoff ab. Man erhält 3,4 g (96 % der

Theorie) 1-(4-Chlorphenyl)-1-/1-(ethylsulfonyl)-1-cyclopropyl7-2-(1,2,4-triazol-1-yl)-1-ethanol vom Schmelzpunkt 215°C.

In analoger Weise und entsprechend den erfindungsgemäßen Verfahren können die in der nachfolgenden Tabelle 1 aufgeführten Verbindungen der Formel (I)

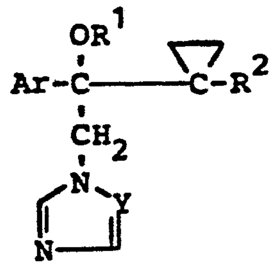

(I)

erhalten werden:

Tabelle 1

| Bsp. Nr. | Ar | $R^1$ | Y | $R^2$ | Schmelzpunkt (°C) |
|---|---|---|---|---|---|
| I-5 | 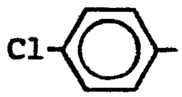 | H | N | 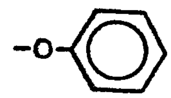 | 139 |
| I-6 | " | H | N | $-S-(CH_2)_3CH_3$ | 129 |
| I-7 | " | H | N | $-SO_2-(CH_2)_3CH_3$ | 136 |
| I-8 | " | H | N | F | 128 |
| I-9 | " | H | N | 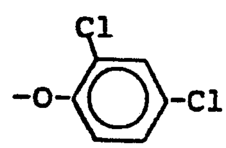 | 167 |
| I-10 | " | H | CH | 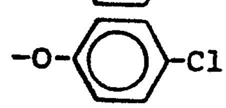 | 211 |
| I-11 | " | H | N | 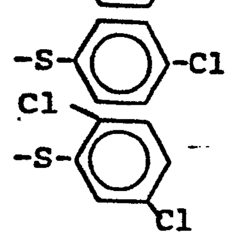 | 137 |
| I-12 | 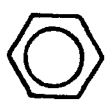 | H | N | | 161 |
| I-13 | 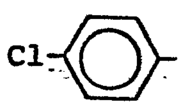 | H | N | 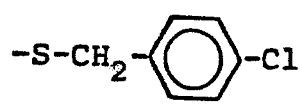 | 183 |
| I-14 | 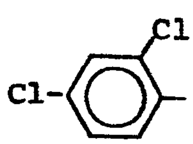 | H | N | 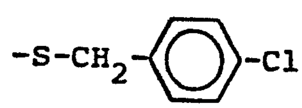 | |
| I-15 | 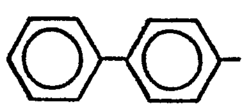 | H | N | 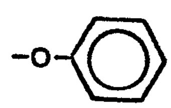 | 121 |

Tabelle 1 (Fortsetzung)

| Bsp. Nr. | Ar | $R^1$ | Y | $R^2$ | Schmelzpunkt (°C) |
|---|---|---|---|---|---|
| I-16 | 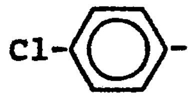 | H | N | $-SCH_3$ | 124 |
| I-17 | " | H | CH | $-SCH_3$ | 159 |
| I-18 | " | H | N | $-SO_2-CH_3$ | 193 |
| I-19 | " | H | N | $-SO_2-C_2H_5$ | 185 |
| I-20 | " | H | CH | $-S-C_2H_5$ | 158 |
| I-21 | " | H | N | $-S-(CH_2)_2-CH_3$ | 131 |
| I-22 | " | H | CH | $-S-(CH_2)_2-CH_3$ | 133 |
| I-23 | " | H | N | $-S-CH_2-CH=CH_2$ | 134 |
| I-24 | " | H | N | 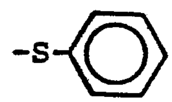 | 141-143 |
| I-25 | " | H | CH | 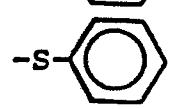 | 145 |
| I-26 | " | H | N | 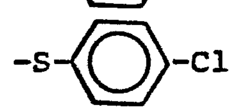 | 94 |
| I-27 | " | H | CH | 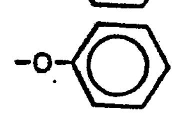 | 208 |
| I-28 | " | H | N | 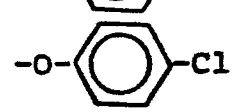 | 165 |

Tabelle 1 (Fortsetzung)

| Bsp. Nr. | Ar | $R^1$ | Y | $R^2$ | Schmelzpunkt (°C) |
|---|---|---|---|---|---|
| I-29 | 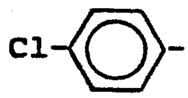 | H | N | 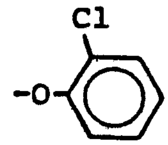 | 119 |
| I-30 | " | H | N | 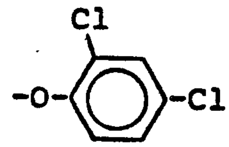 | 181 |
| I-31 | " | H | CH | 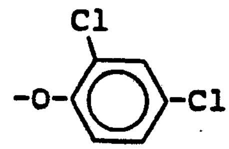 | 213 |
| I-32 | " | H | N | 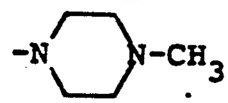 | 122 |
| I-33 | 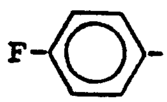 | H | N | F | 148 |
| I-34 | " | H | N | $-SCH_3$ | 113 |
| I-35 | " | H | CH | $-SCH_3$ | 156 |
| I-36 | " | H | N | $-SC_2H_5$ | 126 |
| I-37 | " | H | CH | $-SC_2H_5$ | 161 |
| I-38 | " | H | N | $-S-(CH_2)_3CH_3$ | 130 |
| I-39 | " | H | CH | $-S-(CH_2)_3CH_3$ | 140 |
| I-40 | 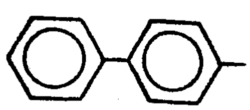 | H | N | 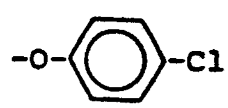 | |

Tabelle 1 (Fortsetzung)

| Bsp. Nr. | Ar | $R^1$ | Y | $R^2$ | Schmelz-punkt (°C) |
|---|---|---|---|---|---|
| I-41 | 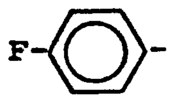 | H | N | 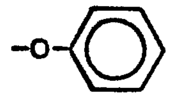 | 171 |
| I-42 | " | H | N | 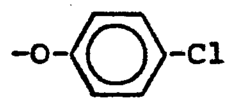 | 144 |
| I-43 | " | H | CH | 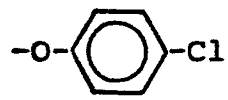 | 184 |
| I-44 | " | H | N | 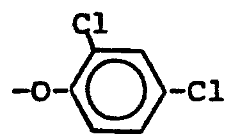 | 141 |
| I-45 | " | H | CH | 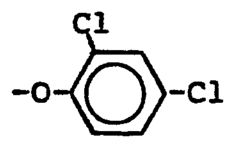 | 227 |
| I-46 | " | H | N | 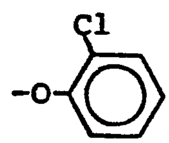 | 122 |
| I-47 | 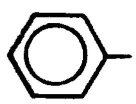 | H | N | $-SC_2H_5$ | 137 |
| I-48 | " | H | CH | $-SC_2H_5$ | 179 |
| I-49 | " | H | N | 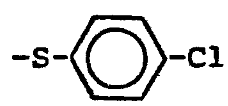 | 144 |
| I-50 | " | H | CH | 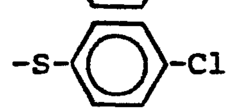 | 168 |
| I-51 | " | H | CH | 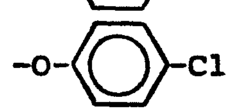 | 177 |
| I-52 | " | H | CH | 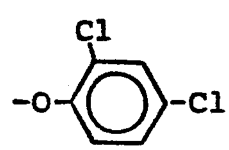 | 245 |

Tabelle 1 (Fortsetzung)

| Bsp. Nr. | Ar | $R^1$ | Y | $R^2$ | Schmelzpunkt (°C) |
|---|---|---|---|---|---|
| I-53 | 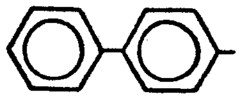 | H | N | F | 123 |
| I-54 | " | H | N | $-SCH_3$ | 163 |
| I-55 | " | H | CH | $-SCH_3$ | 160 |
| I-56 | " | H | N | $-SC_2H_5$ | 155 |
| I-57 | " | H | CH | $-SC_2H_5$ | |
| I-58 | " | H | N | $-S-(CH_2)_3-CH_3$ | 136 |
| I-59 | 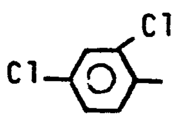 | H | N | $-SC_2H_5$ | 1,5420 |
| I-60 | 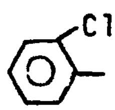 | H | N | $-SC_3H_7$ | 1,5102 |
| I-61 | 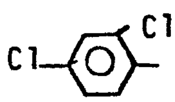 | H | N | $-SC_3H_7$ | 1,5610 |
| I-62 | 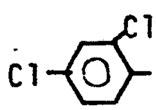 | H | N | $-SC_4H_9$ | 86 |
| I-63 | 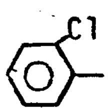 | H | N | $-SC_4H_9$ | 1,5790 |
| I-64 | 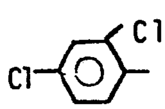 | H | N | $-S-CH_2CH=C(CH_3)_2$ | 1,5698 |
| I-65 | 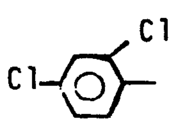 | H | N | $-S-C_3H_7-i$ | 128 |

Tabelle 1 (Fortsetzung)

| Bsp.-Nr. | Ar | $R^1$ | Y | $R^2$ | Schmelzpunkt (°C) |
|---|---|---|---|---|---|
| I-66 | 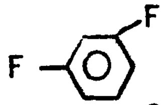 | H | N | $-SC_2H_5$ | 110 |
| I-67 | 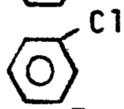 | H | N | $-S-CH_2CH=C(CH_3)_2$ | 1,5598 |
| I-68 | 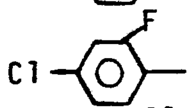 | H | N | $-SC_3H_7$ | 112 |
| I-69 | 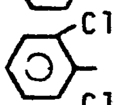 | H | N | $-SC_2H_5$ | 1,5560 |
| I-70 | 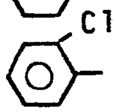 | H | N | $-S-CH_2-C_3H_7$-i | 1,5700 |
| I-71 | 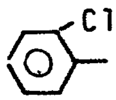 | H | N | $-S-(CH_2)_5CH_3$ | 1,5450 |
| I-72 | 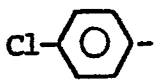 | H | CH | F | 176 |
| I-73 | 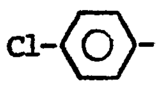 | H | N | $-SO_2-$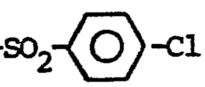 | 166 |
| I-74 | 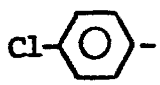 | H | N | $CH_3$<br>$-CH-CH_2-CH_3$ | 104 |
| I-75 | 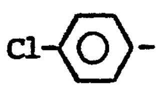 | H | N | $-CH(CH_3)_2$ | 137 |
| I-76 | 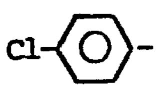 | H | N | $CH_3$<br>$-CH_2-CH-CH_3$ | 147 |
| I-77 | 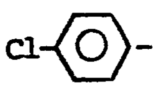 | H | N | $-(CH_2)_5-CH_3$ | 130 |
| I-78 | 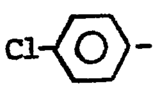 | H | N | $CH_3$<br>$-CH_2-CH_2-CH-CH_3$ | 141 |

Tabelle 1 (Fortsetzung)

| Verb. Nr. | Ar | $R^1$ | Y | $R^2$ | Schmelzpunkt [°C] bzw. Brechungsindex [$n_D^{20}$] |
|---|---|---|---|---|---|
| I-79 | 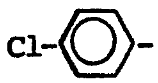 | H | N | $-SO-C_4H_9-n$ | Öl |
| I-80 | 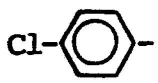 | H | N | $-SO-C_3H_7-n$ | 146 |
| I-81 | 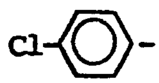 | H | N | 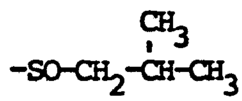 | 113 |
| I-82 | 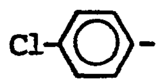 | H | N | 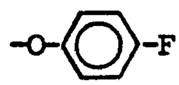 | 147 |
| I-83 | 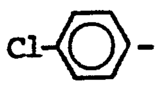 | H | N | 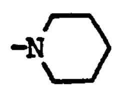 | 130 |
| I-84 | 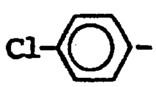 | H | N | $-Cl$ | 149 |
| I-85 | 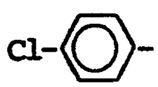 | H | N | $-S-(CH_2)_{11}-CH_3$ | 111 |
| I-86 | 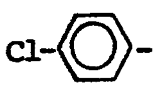 | H | N | $-SO(CH_2)_3-CH_3$ | 153 |
| I-87 | 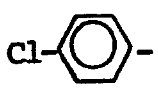 | H | CH | 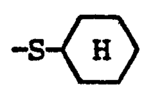 | 191 |
| I-88 | 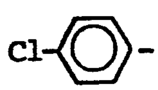 | H | N | 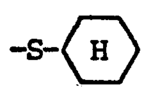 | 142 |
| I-89 | 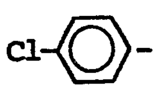 | H | N | $-SO-(CH_2)_{11}-CH_3$ | Öl |
| I-90 | 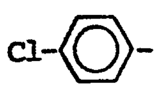 | H | N | $-SO_2-(CH_2)_{11}-CH_3$ | 91 |
| I-91 | 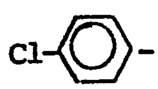 | H | N | $-S-CH_2-CH_2-O$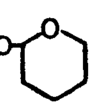 | 75 |

<u>Tabelle 1</u> (Fortsetzung)

| Verb. Nr. | Ar | $R^1$ | Y | $R^2$ | Schmelzpunkt [°C] bzw. Brechungsindex [$n_D^{20}$] |
|---|---|---|---|---|---|
| I-92 | 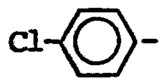 | H | N | $-SO-CH_3$ | 189 (1 Diastereomeres) |
| I-93 | 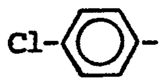 | H | N | $-SO-CH_3$ | 172 (Diastereomeres-Gem.) |
| I-94 | 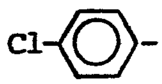 | H | N | $-SO_2-C_3H_7-n$ | 128 |
| I-95 | 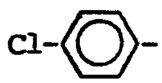 | H | N | $-SO_2-\overset{CH_3}{CH}-C_2H_5$ | 117 |
| I-96 | 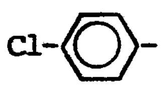 | H | N | $-SO_2-CH(CH_3)_2$ | 159 |
| I-97 | 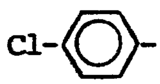 | H | N | $-SO_2-CH_2-CH(CH_3)_2$ | |
| I-98 | 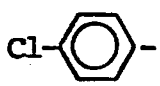 | H | N | $-SO_2-(CH_2)_5-CH_3$ | 140 |
| I-99 | 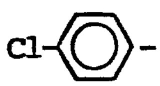 | H | N | $-SO_2-CH_2-CH_2-CH(CH_3)_2$ | 165 |
| I-100 | 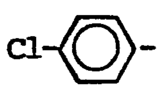 | H | N | $-SO_2-$ 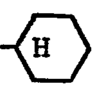 | 176 |
| I-101 | 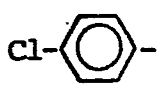 | H | N | $-S-CH_2-CH_2-S-C_2H_5$ | 95 |
| I-102 | 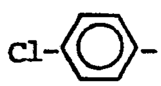 | H | N | $-S-CH_2-COOCH_3$ | 118 |
| I-103 | 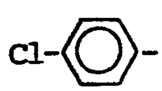 | H | N | $-S-CH_2-CH_2-OH$ | 138 |

Tabelle 1 (Fortsetzung)

| Verb. Nr. | Ar | $R^1$ | Y | $R^2$ | Schmelzpunkt [°C] bzw. Brechungsindex [$n_D^{20}$] |
|---|---|---|---|---|---|
| I-104 | 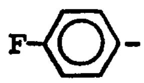 | H | N | $-SO-(CH_2)_3-CH_3$ | Öl |
| I-105 | 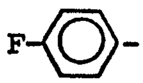 | H | CH | $-S-CH(CH_3)_2$ | 191 |
| I-106 | 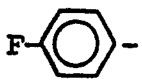 | H | N | $-S-CH(CH_3)_2$ | 155 |
| I-107 | 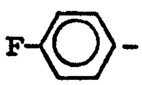 | H | N | -Cl | 121 |
| I-108 | 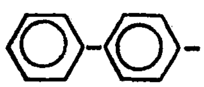 | H | N | $-SO_2CH_3$ | 162 |
| I-109 | 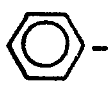 | H | N | $-S-CH(CH_3)_2$ | 136 |
| I-110 | 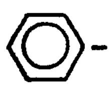 | H | CH | -F | 162 |
| I-111 | 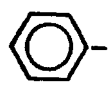 | H | N | -F | 105 |
| I-112 | 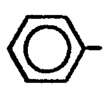 | H | N | $-SCH_3$ | 108 |
| I-113 | 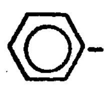 | H | CN | $-SCH_3$ | 167 |
| I-114 | 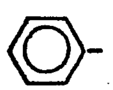 | H | CH | $-S-C_4H_9-n$ | 134 |
| I-115 | 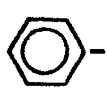 | H | N | $-S-C_4H_9-n$ | 125 |
| I-116 | 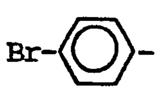 | H | N | -F | 123 |
| I-117 | 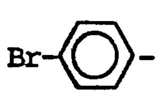 | H | N | $-SC_2H_5$ | 168 |

Tabelle 1 (Fortsetzung)

| Verb. Nr. | Ar | $R^1$ | Y | $R^2$ | Schmelzpunkt [°C] bzw. Brechungsindex [$n_D^{20}$] |
|---|---|---|---|---|---|
| I-118 | 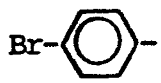 | H | N | -Cl | 127 |
| I-119 | 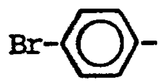 | H | N | $-SCH_3$ | 127 |
| I-120 | 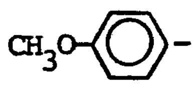 | H | N | -F | 111 |
| I-121 | 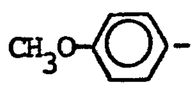 | H | N | $-SC_2H_5$ | 119 |
| I-122 | 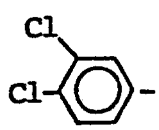 | H | N | 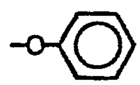 | 139 |
| I-123 | 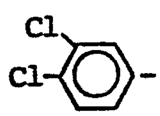 | H | N | $-S-C_4H_9-n$ | 170 |
| I-124 | 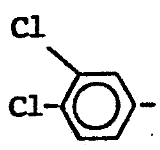 | H | N | $-SO-C_4H_9-n$ | Harz |
| I-125 | 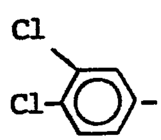 | H | N | $-SO_2-C_4H_9-n$ | 159 |
| I-126 | 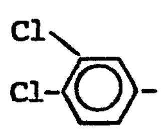 | H | N | $-SCH_3$ | 142 |
| I-127 | 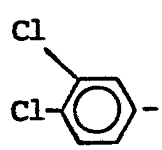 | H | N | $-SO_2CH_3$ | |

Tabelle 1 (Fortsetzung)

| Verb. Nr. | Ar | $R^1$ | Y | $R^2$ | Schmelzpunkt [°C] bzw. Brechungsindex [$n_D^{20}$] |
|---|---|---|---|---|---|
| I-128 | 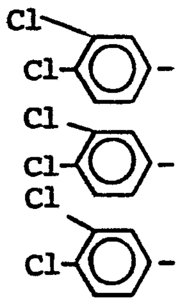 | H | N | $-S-CH_2-CH_2-CH_3$ | |
| I-129 | | H | N | $-S-C_2H_5$ | |
| I-130 | | H | N | $-S-CH(CH_3)_2$ | |
| I-131 | 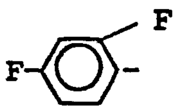 | H | N | $-S-CH_3$ | 83 |
| I-132 | 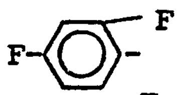 | H | N | $-SO_2CH_3-$ | |
| I-133 | 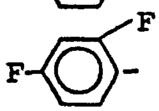 | H | N | -Cl | |
| I-134 | 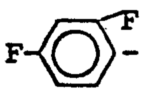 | H | N | 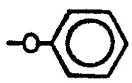 | |
| I-135 | 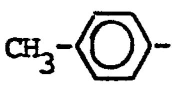 | H | N | -F | 127 |
| I-135 | 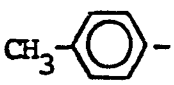 | H | N | $-SC_2H_5$ | 125 |
| I-137 | 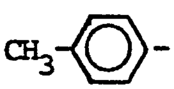 | H | N | 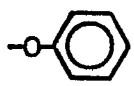 | 146 |
| I-138 | 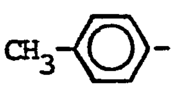 | H | N | $-SCH_3$ | |
| I-139 | 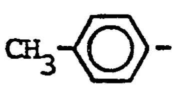 | H | N | $-S-CH(CH_3)_2$ | |
| I-140 | 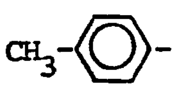 | H | N | $-S-CH_2-CH_2-CH_3$ | |
| I-141 | 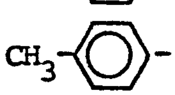 | H | N | $-S-CH_2-CH(CH_3)_2$ | |
| I-142 | 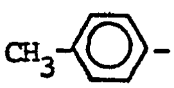 | H | N | $-S-(CH_2)_3-CH_3$ | |

Tabelle 1 (Fortsetzung)

| Bsp. Nr. | Ar | $R^1$ | Y | $R^2$ | Schmelzpunkt /°C/ bzw. Brechungsindex /$n_D^{20}$/ |
|---|---|---|---|---|---|
| I- 143 | 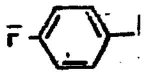 | H | N | $-SO_2CH_3$ | 189 |
| I- 144 | 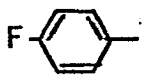 | H | N | $-SO_2-CH(CH_3)_2$ | 177 |
| I- 145 | 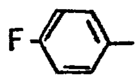 | H | N | $-SO-CH(CH_3)_2$ | 141 |
| I- 146 | 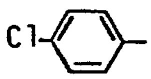 | H | N | -F | 176 |
| I- 147 | 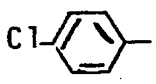 | H | N | $-SO-CH_3$ | 189 |
| I- 148 | 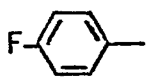 | H | N | -Cl | 109 |
| I- 149 | 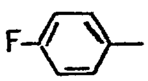 | H | N | -O- | 133 |
| I- 150 | 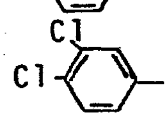 | H | N | $-SO_2-(CH_2)_2-CH_3$ | 104 |
| I- 151 | 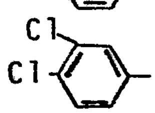 | H | N | $-SO-(CH_2)_2-CH_3$ | 140 |
| I- 152 | 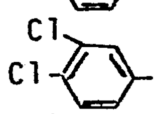 | H | N | $-SO_2-CH_2-CH_3$ | 135 |
| I- 153 | 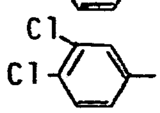 | H | N | $-SO-CH_2-CH_3$ | 112 |
| I- 154 | 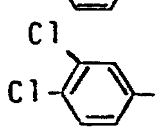 | H | N | $-SO_2-CH(CH_3)_2$ | 137 |
| I- 155 | 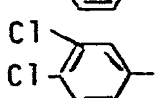 | H | N | $-SO-CH(CH_3)_2$ | 131 |
| I- 156 | 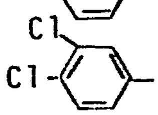 | H | N | $-SO_2-CH_3$ | 160 |
| I- 157 | 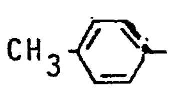 | H | N | -F | 127 |

Tabelle 1 (Fortsetzung)

| Bsp. Nr. | Ar | $R^1$ | Y | $R^2$ | Schmelzpunkt [°C] bzw. Brechungsindex [$n_D^{20}$] |
|---|---|---|---|---|---|
| I-158 | 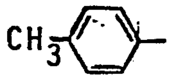 | H | N | $-S-CH-CH_3$ | 125 |
| I-159 | 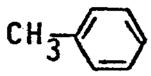 | H | N | $-S-CH_3$ | 116 |
| I-160 | 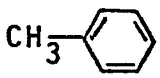 | H | N | $-SO_2-CH(CH_3)_2$ | 149 |
| I-161 | 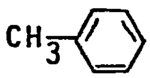 | H | N | $-SO-CH(CH_3)_2$ | 127 |
| I-162 | 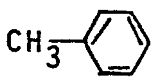 | H | N | $-S(CH_2)_2-CH_3$ | 115 |
| I-163 | 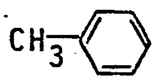 | H | N | $-S-CH_2-CH(CH_3)_2$ | 123 |
| I-164 | 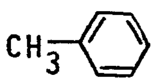 | H | N | $-S-(CH_2)_3-CH_3$ | 107 |
| I-165 | 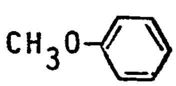 | H | N | -F | 111 |
| I-166 | 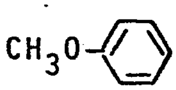 | H | N | $-S-CH_2-CH_3$ | 119 |
| I-167 | 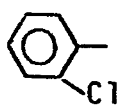 | H | N | -S- 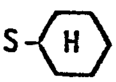 | 1,5630 |
| I-168 | 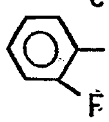 | H | N | $-SC_3H_7$ | 75 |
| I-169 | 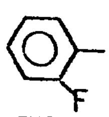 | H | N | $-SC_4H_9$ | 75 |
| I-170 | 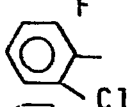 | H | N | $-S-CH(CH_3)_2$ | 1,5630 |
| I-171 | 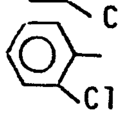 | H | N | $-S-C(CH_3)_3$ | 1,5660 |
| I-172 | 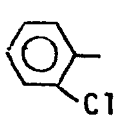 | H | N | $CH_3$<br>$-S-CH-C_2H_5$ | 1,5582 |

Tabelle 1 (Fortsetzung)

| Bsp.-Nr. | Ar | $R^1$ | Y | $R^2$ | Schmelzpunkt [°C] bzw. Brechungsindex [$n_D^{20}$] |
|---|---|---|---|---|---|
| I-173 | 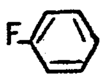 | H | N | $-S-CH(CH_3)_2$ | 106 |
| I-174 | | H | N | $-S-C_3H_7$ | 110 |
| I-175 | | H | N | $-S-C_3H_7$ | 1,5420 |
| I-176 | 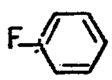 | H | N | $-S-CH(CH_3)_2$ | 118 |
| I-177 | | H | N | $-S-CH(CH_3)_2$ | 80 |

0180850

**Weitere Herstellungsbeispiele für die Ausgangsstoffe (II)**

Beispiel (II-2)

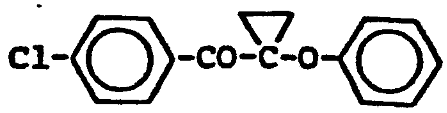

(II-2)

Zu einer Lösung von 29,6 g (0,1 Mol) 4-Chlorphenyl-(1-brom-3-chlorpropyl)-keton und 9,4 g Phenol in 60 ml Dimethylformamid gibt man 14 g Kaliumcarbonat und erwärmt drei Stunden auf 50°C. Danach tropft man bei Raumtemperatur 8,4 g Kaliumhydroxid in 40 ml Methanol zu und erwärmt drei Stunden auf 60°C. Man engt im Vakuum ein und nimmt in einem Wasser/Methylenchlorid-Gemisch auf. Die organische Phase wird abgetrennt, mit Wasser gewaschen, über Natriumsulfat getrocknet und im Vakuum eingeengt. Der Rückstand wird im Hochvakuum destilliert. Man erhält 20,7 g (76,1 % der Theorie) 1-(4-Chlorbenzoyl)-1-phenoxy-cyclopropan vom Siedepunkt $K_{p\ 0,15}$ = 170-80°C.

Beispiel (II-3)

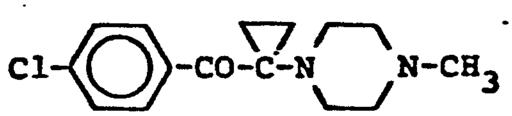

(II-3)

Zu einer Mischung aus 10,4 g N-Methylpiperazin und 14 g Kaliumcarbonat in 30 ml Dimethylformamid tropft man eine Lösung von 29,6 g (0,1 Mol) 4-Chlorphenyl-(1-brom-3-chlorpropyl)-keton in 30 ml Dimethylformamid bei Raumtemperatur zu. Man läßt drei Stunden nachrühren und tropft dann eine Lösung von 9 g Kaliumhydroxid in 30 ml Methanol zu. Das Reaktionsgemisch wird eine Stunde bei 50°C nachgerührt und im Vakuum eingeengt. Der Rückstand wird in Essigester/Wasser aufgenommen, die organische Phase abgetrennt, mit Wasser gewaschen, über Natriumsulfat getrocknet und im Vakuum eingeengt. Der Rückstand wird über Kieselgel mit Chloroform/Ethanol (97:3) chromatographiert. Man erhält 14 g (50,3 % der Theorie) 1-(4-Chlorbenzoyl)-1-(4-methyl-piperazin-1-yl)-cyclopropan als rötliches Öl, das direkt weiter eingesetzt werden kann.

Beispiel (II-4)

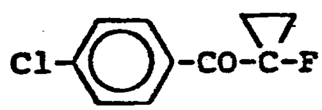

(II-4)

Man löst 20 g (0,085 Mol) 4-Chlorphenyl-(1-fluor-3-chlorpropyl)-keton in 150 ml tert.-Butanol und versetzt portionsweise mit 15 g Kalium-tert.-butylat. Man läßt 2 Stunden bei 40°C nachrühren und engt im Vakuum ein. Der Rückstand wird in Methylenchlorid und Wasser aufgenommen. Man trennt die organische Phase ab,

trocknet über Natriumsulfat und engt im Vakuum ein. Der Rückstand wird im Hochvakuum destilliert. Man erhält 14,4 g (85 % der Theorie) 1-(4-Chlorbenzoyl)-1-fluor-cyclopropan vom Siedepunkt $Kp_{0,1}$ = 75°C.

Herstellung des Ausgangsproduktes

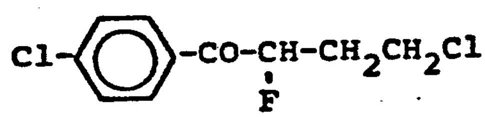

Eine Mischung aus 68 g 4-Chlorphenyl-(1-brom-3-chlor-propyl)-keton, 27,5 g getrocknetem Kaliumfluorid, 11 g 18-Krone-6 (1,4,7,10,13,16-Hexaoxacyclooctadekan der Formel

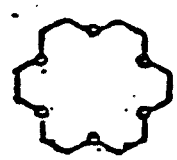

)

und 200 ml trockenem Benzol wird 8 Stunden unter Rückfluß erhitzt. Danach gibt man 200 ml Wasser zu, trennt die organische Phase ab, wäscht mehrmals mit Wasser, trocknet über Natriumsulfat und engt im Vakuum ein. Der Rückstand wird mit Leichtbenzin unter Zusatz von wenig Toluol verrührt. Nach Absaugen und Trocknen erhält man 28,5 g (53 % der Theorie) 4-Chlorphenyl-(1-fluor-3-chlor-propyl)-keton vom Schmelzpunkt 53°C.

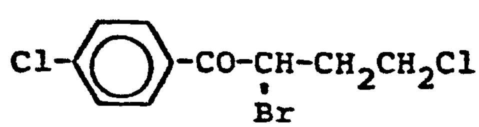

Eine Lösung von 483 g (2,23 Mol) 4-Chlorphenyl-3-chlor-propyl-keton in 1200 ml Methylenchlorid wird bei 20°C tropfenweise mit einer Lösung von 358 g Brom in 350 ml Methylenchlorid versetzt. Man läßt eine Stunde nachrühren und engt damm im Vakuum ein. Der Rückstand wird mit 300 ml Petrolether versetzt und bis zur Vollständigkeit der Kristallisation gerührt. Man kühlt auf 10°C ab und saugt den Niederschlag ab. Man erhält 582 g (88,3 % der Theorie) 4-Chlorphenyl-(1-brom-3-chlor-propyl)-keton vom Schmelzpunkt 73°C.

Entsprechend der Beispiele (II-1) bis (II-4) und gemäß den angegebenen Verfahrensbedingungen können die in der nachfolgenden Tabelle 2 aufgeführten Vorprodukte der Formel (II)

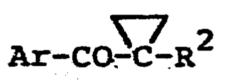 (II)

erhalten werden:

0180850

| Bsp. Nr. | Ar | $R^2$ | Physikal. Konstante |
|---|---|---|---|
| II-5 | 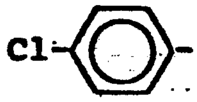 | $-S-(CH_2)_3CH_3$ | $Kp_{0,1}$ = 133°C |
| II-6 | 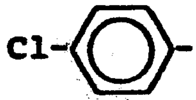 | $-S-CH_3$ | $Kp_{0,01}$ = 120°C |
| II-7 | 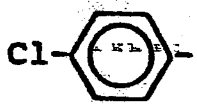 | $-C(CH_3)_3$ | zähfl. Öl |
| II-8 | 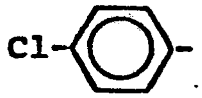 | 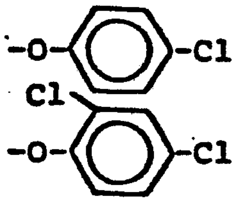 | $Kp_{0,1}$ = 160°C |
| II-9 | 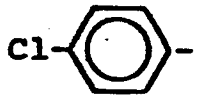 | | $Kp_{0,1}$ = 173°C |
| II-10 | 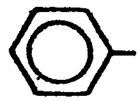 | 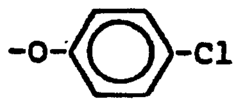 | $Kp_{0,01}$ = 143°C |
| II-11 | 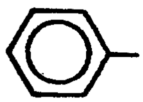 | 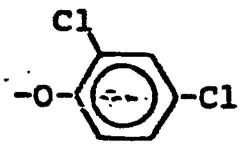 | $Kp_{0,01}$ = 146°C |
| II-12 | 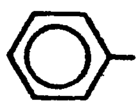 | 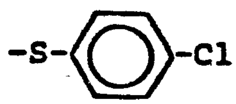 | Fp. = 86°C |
| II-13 | 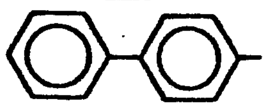 | $-SCH_3$ | Fp. = 98°C |
| II-14 | 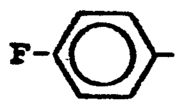 | $-SCH_3$ | $Kp_{0,1}$ = 102°C |
| II-15 | 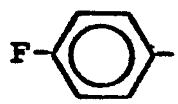 | 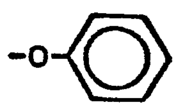 | $Kp_{0,1}$ = 125-130°C |
| II-16 | 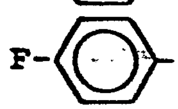 | 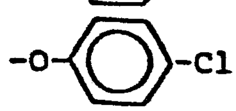 | $Kp_{0,1}$ = 145-150°C |
| II-17 | 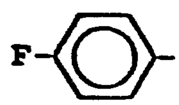 | 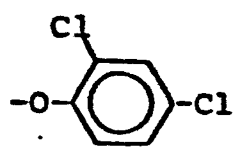 | $Kp_{0,1}$ = 165-173°C |

| Bsp. Nr. | Ar | $R^2$ | Physikal. Konstante |
|---|---|---|---|
| II-18 | 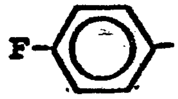 | $-S-C_2H_5$ | $Kp_{0,07}$ = 95°C |
| II-19 | 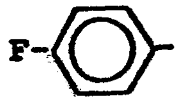 | $-S-(CH_2)_3CH_3$ | $Kp_{0,1}$ = 113-116°C |
| II-20 | 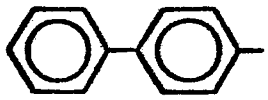 | $-S-C_2H_5$ | Fp. = 86°C |
| II-21 | 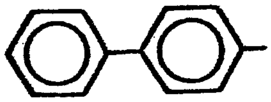 | 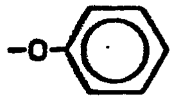 | Fp. = 77°C |
| II-22 | 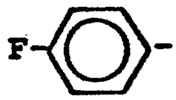 | 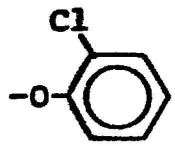 | $Kp_{0,1}$ = 147-155°C |
| II-23 | 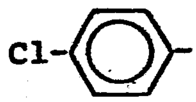 | 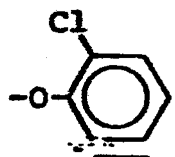 | $Kp_{0,07}$ = 154°C |
| II-24 | 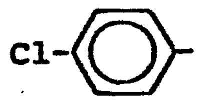 | 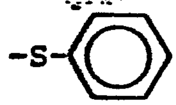 | $Kp_{0,1}$ = 163-170°C |
| II-25 | 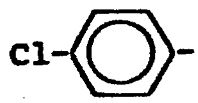 | 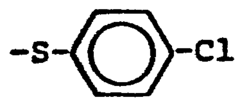 | Fp. = 104°C |
| II-26 | 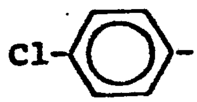 | $-S-CH_2-CH=CH_2$ | $Kp_{0,06}$ = 122°C |
| II-27 | 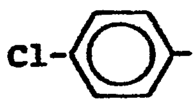 | $-C_3H_7-n$ | $Kp_{0,15}$ = 118°C |
| II-28 | 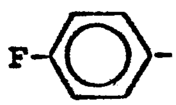 | F | $Kp_{0,07}$ = 55°C |
| II-29 | 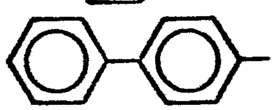 | F | $Kp_{0,15}$ = 138°C/Fp. 73° |
| II-30 | 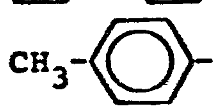 | F | $Kp_{0,1}$ = 69°C |

| Bsp. Nr. | Ar | $R^2$ | Physikal. Konstante |
|---|---|---|---|
| II-31 | 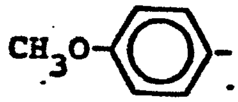 | F | $Kp_{0,1}$ = 98°C |
| II-32 | 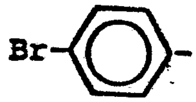 | F | $Kp_{0,1}$ = 88°C |

Entsprechend Beispiel 1 und gemäß den angegebenen Verfahrensbedingungen können die in der nachfolgenden Tabelle 3 aufgeführten Zwischenprodukte der Formel (IV)

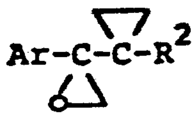

(IV)

erhalten werden:

| Bsp. Nr. | Ar | $R^2$ | Physikal. Konstante |
|---|---|---|---|
| IV-2 | 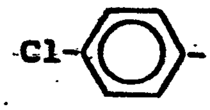 | 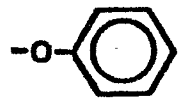 | zähfl. Öl |
| IV-3 | " | $-S-(CH_2)_3CH_3$ | zähfl. Öl |
| IV-4 | " | F | $Kp_{0,2}$ = 83°C |
| IV-5 | " | $-SCH_3$ | Öl |
| IV-6 | " | $-SC_2H_5-$ | Öl |
| IV-7 | " | $-S-(CH_2)_3-CH_3$ | Öl |
| IV-8 | " | $-S-CH_2-CH=CH_2$ | Öl |
| IV-9 | " | $-S-(CH_2)_2-CH_3$ | Öl |
| IV-10 | " | 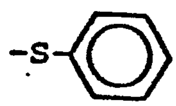 | Öl |
| IV-11 | " | 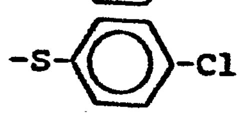 | Öl |
| IV-12 | " | 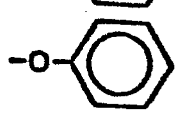 | Öl |
| IV-13 | " | 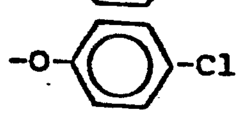 | Öl |
| IV-14 | " | 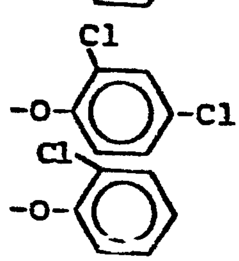 | Fp. 81°C |
| IV-15 | " |  | Fp. 97°C |

| Bsp. Nr. | Ar | $R^2$ | Physikal. Konstante |
|---|---|---|---|
| IV-16 | 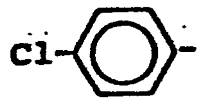 | F | $Kp_{0,2}$ = 83°C |
| IV-17 | " | 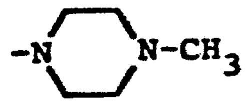 | Öl |
| IV-18 | 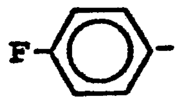 | $-SCH_3$ | Öl |
| IV-19 | " | $-SC_2H_5$ | Öl |
| IV-20 | " | $-S-(CH_2)_3-CH_3$ | Öl |
| IV-21 | " | 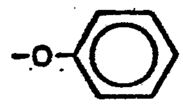 | Öl |
| IV-22 | " | 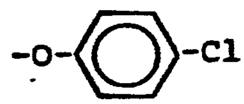 | Öl |
| IV-23 | " | 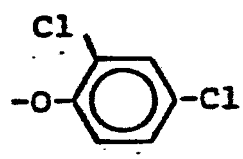 | Fp. 72°C |
| IV-24 | " | 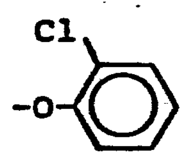 | Öl |
| IV-25 | " | F | Öl |
| IV-26 | 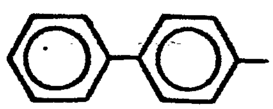 | $-SCH_3$ | Öl |
| IV-27 | " | $-SC_2H_5$ | Öl |
| IV-28 | " | $-S-(CH_2)_3-CH_3$ | Öl |

| Bsp. Nr. | Ar | $R^2$ | Physikal. Konstante |
|---|---|---|---|
| IV-29 | 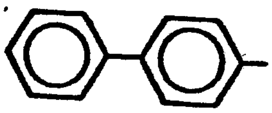 | 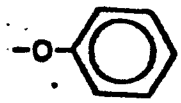 | Fp. 94°C |
| IV-30 | " | 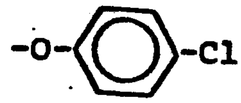 | Fp. 91°C |
| IV-31 | " | F | Öl |
| IV-32 | 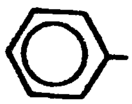 | $-SC_2H_5-$ | Öl |
| IV-33 | " | 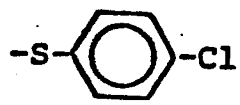 | Öl |
| IV-34 | " | 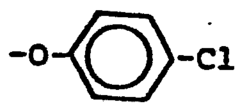 | Öl |
| IV-35 | " | 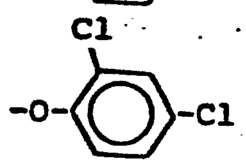 | Fp. 62°C |

Entsprechend Beispiel 2 und gemäß den angegebenen Verfahrensbedingungen können die in der nachfolgenden Tabelle 4 aufgeführten Zwischenprodukte der Formel (VI)

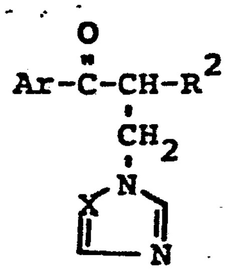

(VI)

erhalten werden:

| Bsp. Nr. | Ar | X | $R^2$ | physikal. Konstante |
|---|---|---|---|---|
| VI-2 | 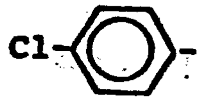 | N | 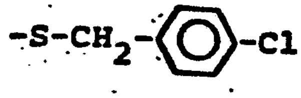 | Fp. 102°C |
| VI-3 | 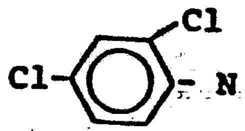 | N | ″ | Fp. 69°C |
| VI-4 | 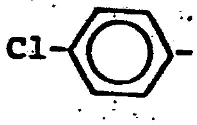 | N | 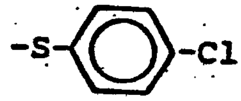 | Fp. 102°C |
| VI-5 | 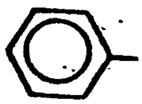 | N | 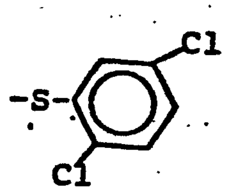 | Fp. 117°C |
| VI-6 | 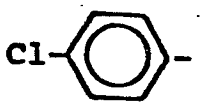 | N | ″ | Fp. 148°C |
| VI-7 | 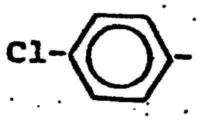 | CH | 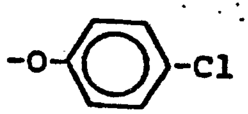 | Fp. 153°C |
| VI-8 | 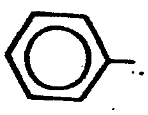 | N | 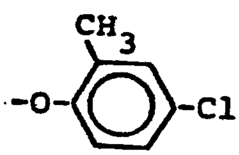 | Fp. 139°C |
| VI-9 | 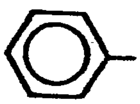 | N | 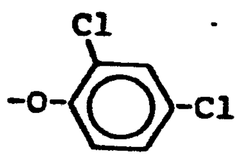 | Fp. 156°C |
| VI-10 | 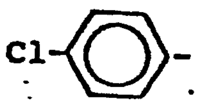 | N | 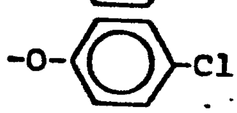 | Fp. 128°C |

| Bsp. Nr. | Ar | Y | $R^2$ | Schmelzpunkt (°C) |
|---|---|---|---|---|
| VI-11 | 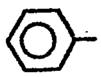 | N | -S- 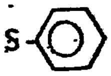 | 102 |
| VI-12 | 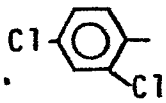 | N | $-S-C_2H_5$ | 50 |
| VI-13 | 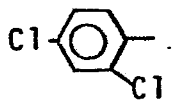 | N | $-S-C_3H_7$ | 1,5632 |
| VI-14 | 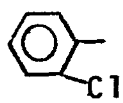 | N | $-S-C_2H_5$ | 62 |
| VI-15 | 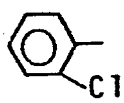 | N | $-S-C_3H_7$ | 58 |
| VI-16 | 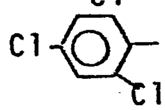 | N | $-S-C_4H_9$ | 1,5742 |
| VI-17 | 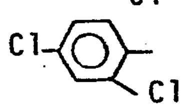 | N | $-S-CH(CH_3)_2$ | 1,5762 |

Verwendungsbeispiele

In dem in-vitro-Test werden die nachstehend angegebenen, aus der EP-OS 0 044 605 bekannten Verbindungen als Vergleichssubstanzen eingesetzt:

(A)

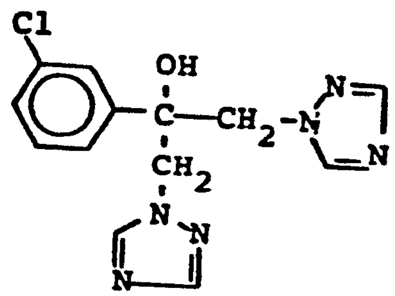

(B)

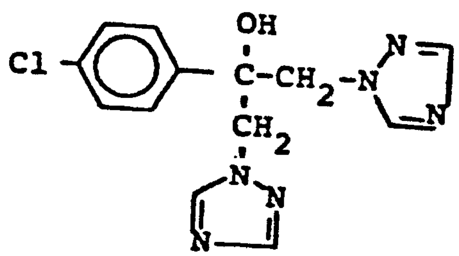

(C)

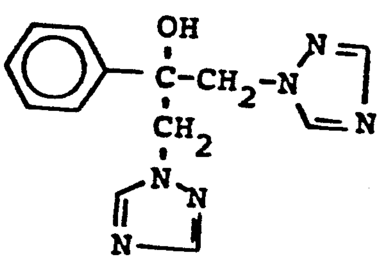

## Beispiel A

### Antimykotische in-vitro-Wirksamkeit

Versuchsbeschreibung:

Die in-vitro-Prüfungen wurden im Reihenverdünnungstest mit Keiminokula von durchschnittlich 5 x $10^3$ bis $10^4$ Keimen/ml Substrat durchgeführt. Als Nährmedium dienten

a) für Dermatophyten und Schimmelpilze:
Sabourand's milieu d'épreuve

b) für Hefen:
Fleischextrakt-Traubenzucker-Bouillon.

Die Bebrütungstemperatur betrug 28 bis 37°C, die Bebrütungsdauer lag bei 24 bis 96 Stunden bei Hefen und 96 Stunden bei Dermatophyten und Schimmelpilzen.

In diesem Test zeigten z.B. die erfindungsgemäßen Verbindungen 1, 2, 3, 4, 5, 6, 7, 11 und 12 eine bessere antimykotische Wirkung als die aus dem Stand der Technik bekannten Verbindungen (A), (B) und (C).

Tabelle A

Antimykotische in-vitro-Wirksamkeit

| Wirkstoff | MHK-Werte in mg/ml Nährmedium Tricho-phyton mentagr. | Micro-sporum canis | Candida albi-cans | Toru-lopsis glabrata | Asper-gillus fumigatus |
|---|---|---|---|---|---|
| (A) (bekannt) | 32 | - | > 64 | > 64 | > 64 |
| (B) (bekannt) | 16 | - | 2 | 64 | > 64 |
| (C) (bekannt) | 64 | - | 64 | > 64 | > 64 |
| Verbindungen gemäß Herstellungsbeispiel | | | | | |
| I-1 | < 1 | 4 | < 1 | < 1 | 4 |
| I-2 | < 1 | 32 | < 1 | 4 | 4 |
| I-3 | 2 | 64 | < 1 | 64 | 32 |
| I-4 | < 1 | 2 | < 1 | 4 | 2 |
| I-5 | < 1 | > 64 | < 1 | < 1 | 64 |
| I-6 | 2 | > 64 | < 1 | 16 | 64 |
| I-7 | < 1 | 2 | 4 | < 1 | < 1 |
| I-11 | 2 | 16 | < 1 | 32 | > 64 |
| I-12 | 4 | 64 | < 1 | 8 | > 64 |

**Beispiel B**

**Antimykotische in-vivo-Wirksamkeit (oral) bei Mäusecandidose**

Versuchsbeschreibung:

Mäuse vom Typ SPF-$CF_1$ wurden intravenös mit 1-2 x $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 10 bis 100 mg/kg Körpergewicht der Präparate oral behandelt.

Ergebnis

Unbehandelte Tiere starben am 3. bis 6. Tag post infektionem. Die Überlebensrate am 6. Tag post infektionem betrug bei unbehandelten Kontrolltieren etwa 5 %.

In diesem Test zeigen z.B. die erfindungsgemäßen Verbindungen 1, 2, 3, 4, 5, 6, 7 und 13 gute bis sehr gute Wirkung, d.h. 60 % Überlebende am 6. Tag p.i.

**Tabelle B**

Antimykotische in-vivo-Wirkung (oral) bei Mäusecandidose

| Wirkstoff | Wirkung |
|---|---|
| I-1 | +++++ |
| I-2 | +++++ |
| I-3 | +++++ |
| I-4 | +++ |
| I-5 | +++++ |
| I-6 | ++++ |
| I-7 | +++++ |
| I-13 | +++++ |

**Zeichenerklärung**

| | | |
|---|---|---|
| +++++ | = sehr gute Wirkung | = 90 % Überlebende am 6. Tag p.i. |
| ++++ | = gute Wirkung | = 80 % Überlebende am 6. Tag p.i. |
| +++ | = Wirkung | = 60 % Überlebende am 6. Tag p.i. |
| ++ | = schwache Wirkung | = 40 % Überlebende am 6. Tag p.i. |
| + | = Spur Wirkung | = unter 40 % Überlebende am 6. Tag p.i. |
| k.W. | | = kein Unterschied zur unbehandelten Infektionskontrolle. |

0180850

Patentansprüche

1. Azolylmethyl-cyclopropyl-carbinol-Derivate der allgemeinen Formel (I)

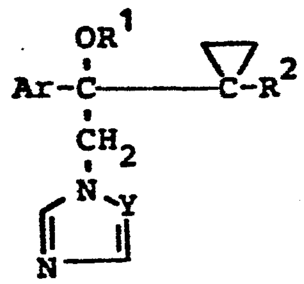

(I)

in welcher

Ar für gegebenenfalls substituiertes Aryl oder gegebenenfalls substituiertes Hetaryl steht,

$R^1$ für Wasserstoff, Alkyl, Alkenyl, Alkinyl, Trialkylsilyl, gegebenenfalls substituiertes Phenylalkyl oder den Acyl-Rest steht,

$R^2$ für Halogen, Cyano, Thiocyanato, Alkylcarbonyloxy, Alkylcarbonylthio oder die Gruppierungen $-X-R^3$ und $-NR^4R^5$ steht, sowie auch für Wasserstoff steht, wenn Ar für gegebenenfalls substituiertes Hetaryl steht,

$R^3$ für Alkyl, Cycloalkyl, Alkenyl, Hydroxyalkyl, Alkylthioalkyl, Carboxyalkyl, Alkoxycarbonylalkyl, gegebenenfalls substituiertes Aryl, gegebenenfalls substituiertes Aralkyl oder den Rest der Formel

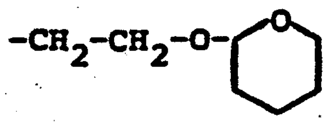

steht,

$R^4$ und $R^5$ gleich oder verschieden sind und für Wasserstoff oder Alkyl stehen, sowie gemeinsam mit dem Stickstoffatom, an das sie gebunden sind, für einen gegebenenfalls substituierten cycloaliphatischen Ring stehen, der gegebenenfalls weitere Heteroatome enthält,

X für Sauerstoff, Schwefel, die SO- oder $SO_2$-Gruppe steht und

Y für ein Stickstoffatom oder die CH-Gruppe steht,

sowie deren Säureadditionssalze bei der Verwendung zur Bekämpfung von Krankheiten, insbesondere Mykosen.

2. Verbindungen der allgemeinen Formel (I) gemäß Anspruch 1, in welcher

Ar für gegebenenfalls einfach oder mehrfach, gleich oder verschieden substituiertes Phenyl steht, wobei als Substituenten genannt seien: Halogen; Alkyl, Alkoxy und Alkylthio mit jeweils 1 bis 4 Kohlenstoffatomen; Halogenalkyl, Halogenalkoxy und Halogenalkylthio mit jeweils 1 bis 2 Kohlenstoffatomen und 1 bis 5 gleichen oder verschiedenen Halogenatomen, wie Fluor- und Chloratomen,

sowie jeweils gegebenenfalls durch Alkyl mit 1 oder 2 Kohlenstoffatomen und/oder Halogen substituiertes Phenyl oder Phenoxy steht, weiterhin für Naphthyl sowie für einen gegebenenfalls einfach oder mehrfach, gleich oder verschieden substituierten 5- oder 6-gliedrigen Heteroaromaten mit Stickstoff, Sauerstoff und/oder Schwefel als Heteroatome steht, wobei als Substituenten die obengenannten Phenylsubstituenten in Frage kommen;

$R^1$ für Wasserstoff, geradkettiges oder verzweigtes Alkyl mit 1 bis 4 Kohlenstoffatomen, geradkettiges oder verzweigtes Alkenyl und Alkinyl mit jeweils 2 bis 4 Kohlenstoffatomen, für Trialkylsilyl mit 1 bis 4 Kohlenstoffatomen in jedem Alkylteil, Alkylcarbonyl mit 1 bis 4 Kohlenstoffatomen im Alkylteil, sowie für gegebenenfalls einfach oder mehrfach, gleich oder verschieden substituiertes Phenylalkyl mit 1 bis 2 Kohlenstoffatomen in Alkylteil steht, wobei als Substituenten die bei Ar bereits genannten Phenylsubstituenten in Frage kommen;

$R^2$ für Fluor, Chlor, Brom, Cyano, Thiocyanato, Alkylcarbonyloxy oder Alkylcarbonylthio mit jeweils 1 bis 4 Kohlenstoffatomen im Alkylteil oder für die Gruppierungen $-X-R^3$ und $-NR^4R^5$ steht, sowie auch für Wasserstoff steht, wenn Ar einen gegebenenfalls substituierten Heteroaromaten bedeutet,

$R^3$ für geradkettiges oder verzweigtes Alkyl mit 1 bis 12 Kohlenstoffatomen, Cycloalkyl mit 5 bis 7 Kohlenstoffatomen, geradkettiges oder verzweigtes Alkenyl mit 2 bis 12 Kohlenstoffatomen, geradkettiges oder verzweigtes Alkinyl mit 2 bis 12 Kohlenstoffatomen, Hydroxyalkyl mit 1 bis 12 Kohlenstoffatomen, Alkylthioalkyl mit 1 bis 4 Kohlenstoffatomen in der Alkylthiogruppe und 1 bis 4 Kohlenstoffatomen in der Alkylgruppe, Carboxyalkyl mit 1 bis 12 Kohlenstoffatomen im Alkylteil, Alkoxycarbonylalkyl mit 1 bis 4 Kohlenstoffatomen im Alkoxyteil und 1 bis 4 Kohlenstoffatomen im Alkylteil, sowie für jeweils gegebenenfalls einfach bis dreifach, insbesondere einfach oder zweifach, gleichartig oder verschieden substituiertes Phenyl oder Benzyl steht, wobei als Substituenten die oben bei Ar bereits genannten besonders bevorzugten Phenylsubstituenten in Frage kommen, ode.

$R^3$ für den Rest der Formel

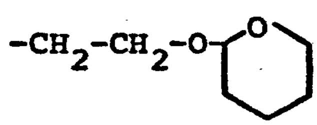

steht,

$R^4$ und $R^5$ die gleich oder verschieden sein können, für Wasserstoff, geradkettiges oder verzweigtes Alkyl mit 1 bis 4 Kohlenstoffatomen, sowie gemeinsam mit dem Stickstoff, Alkyl, an das sie gebunden sind, für einen gegebenenfalls

durch Alkyl mit 1 bis 4 Kohlenstoffatomen substituierten 5- oder 6-gliedrigen Ring mit gegebenenfalls Sauerstoff, Schwefel oder Stickstoff als weitere Heteroatome stehen,

X für Sauerstoff, Schwefel, die SO- oder $SO_2$-Gruppe und

Y für ein Stickstoffatom oder die CH-Gruppe steht

bei der Verwendung zur Bekämpfung von Krankheiten, insbesondere Mykosen.

3. Verbindungen der allgemeinen Formel (I) gemäß Anspruch 1, in welcher

Ar für gegebenenfalls einfach bis dreifach, insbesondere einfach oder zweifach, gleich oder verschieden substituiertes Phenyl steht, wobei als Substituenten genannt seien: Fluor, Chlor, Methyl, Isopropyl, tert.-Butyl, Methoxy, Methylthio, Trifluormethyl, Trifluormethoxy, Trifluormethylthio, sowie jeweils gegebenenfalls durch Fluor, Chlor und/oder Methyl substituiertes Phenyl oder Phenoxy; weiterhin für Naphthyl sowie für jeweils gegebenenfalls einfach oder zweifach, gleich oder verschieden substituiertes Furyl, Thienyl, Pyridinyl oder Pyrimidinyl steht, wobei als Substituenten die obengenannten Phenylsubstituenten in Frage kommen;

0180850

$R^1$ für Wasserstoff, Methyl, Ethyl, n-Propyl, Isopropyl, n-Butyl, Isobutyl, Allyl, Propargyl, Trimethylsilyl, Methylcarbonyl, Ethylcarbonyl, n-Propyl-carbonyl, Isopropylcarbonyl, n-Butylcarbonyl, Isobutylcarbonyl, sowie für gegebenenfalls einfach bis dreifach, insbesondere einfach oder zweifach, gleich oder verschieden substituiertes Benzyl steht, wobei als Substituenten die bei Ar bereits genannten Phenylsubstituenten in Frage kommen und

$R^2$ für Fluor, Chlor, Brom, Cyano, Thiocyanato, Methylcarbonyloxy, Ethylcarbonyloxy, n-Propylcarbonyloxy, Isopropylcarbonyloxy, n-Butylcarbonyloxy, Isobutylcarbonyloxy, Methylcarbonylthio, Ethylcarbonylthio, n- oder i-Propylcarbonylthio, n- oder i-Butylcarbonylthio oder für die Gruppierungen $-X-R^3$ und $-NR^4R^5$ steht, sowie auch für Wasserstoff steht, wenn Ar für einen gegebenenfalls substituierten Heteroaromaten steht;

$R^3$ für geradkettiges oder verzweigtes Alkyl mit 1 bis 12 Kohlenstoffatomen, Cycloalkyl mit 5 bis 7 Kohlenstoffatomen, geradkettiges oder verzweigtes Alkenyl mit 2 bis 12 Kohlenstoffatomen, geradkettiges oder verzweigtes Alkinyl mit 2 bis 12 Kohlenstoffatomen, Hydroxyalkyl mit 1 bis 12 Kohlenstoffatomen, Alkinylthioalkyl mit 1 bis 4 Kohlenstoffatomen in der Alkylthiogruppe und 1 bis 4 Kohlenstoffatomen in der Alkylgruppe, Carboxyalkyl mit 1 bis 12 Kohlenstoffatomen im Alkylteil, Alkoxycarbonylalkyl mit 1 bis 4 Kohlenstoffatomen im Alkoxyteil und 1 bis 4 Kohlenstoffatomen im Alkylteil, sowie für jeweils gegegebenenfalls einfach bis dreifach, insbesondere einfach oder zweifach, gleichartig oder verschieden substituiertes Phenyl oder Benzyl steht, wobei als Substituenten die oben bei Ar bereits genannten besonders bevorzugten Phenylsubstituenten in Frage kommen, oder

$R^3$ für den Rest der Formel

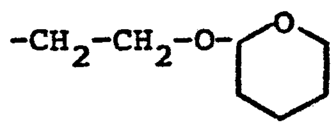

steht,

$R^4$ und $R^5$ die jedoch gleich oder verschieden sein können, für Wasserstoff, Methyl, Ethyl, n-Propyl, Isopropyl, n-Butyl oder Isobutyl stehen, sowie auch für jeweils gegebenenfalls durch Methyl, Ethyl, Methylcarbonyl oder Ethylcarbonyl substituiertes Piperidin, Piperazin oder Morpholin stehen;

X für Sauerstoff, Schwefel, die SO- oder $SO_2$-Gruppe steht und

Y für ein Stickstoffatom oder die CH-Gruppe steht

bei der Verwendung zur Bekämpfung von Krankheiten, insbesondere Mykosen.

4. 1-(4-Chlorphenyl)-1-[1-(ethylsulfinyl)-1-cyclopropyl]-2-(1,2,4-triazol-1-yl)-1-ethanol bei der Verwendung zur Bekämpfung von Krankheiten, insbesondere Mykosen.

5. 1-(4-Chlorphenyl)-1-[1-(n-butylsulfinyl)-1-cyclopropyl]-2-(1,2,4-triazol-1-yl)-1-ethanol bei der Verwendung zur Bekämpfung von Krankheiten, insbesondere Mykosen.

6. Arzneimittel enthaltend mindestens eine Verbindung der allgemeinen Formel (I) gemäß den Ansprüchen 1 bis 3.

7. Verfahren zur Herstellung von Arzneimitteln gemäß Anspruch 6, dadurch gekennzeichnet, daß man den oder die Wirkstoffe gegebenenfalls unter Verwendung von üblichen Hilfs- und Trägerstoffen in eine geeignete Applikationsform überführt.